# EUROPEAN PATENT APPLICATION

(11) **EP 3 335 719 A1**
(43) Date of publication of application: **20.06.2018**
(21) Application number: 16204038.0
(22) Date of filing: 14.12.2016
(51) Int. Cl.: A61K 38/00, C07K 14/33, C12N 9/52

(54) **NOVEL RECOMBINANT BOTULINUM NEUROTOXINS WITH A STABILIZED LIGHT CHAIN**

(71) Applicant: Merz Pharma GmbH & Co. KGaA, 60318 Frankfurt am Main (DE)
(72) Inventor: Frevert, Jürgen, 10625 Berlin (DE); Hofmann, Fred, 14480 Potsdam (DE); López de la Paz, Manuela, 65835 Liederbach am Taunus (DE); Scheps, Daniel, 14471 Potsdam (DE); Jurk, Marcel, 13403 Berlin (DE)
(74) Representative: Hackenberg, Julia

(57) **Abstract**

This invention relates to novel recombinant botulinum neurotoxins serotype A exhibiting a higher thermostability and/or a higher oxidative resistance and/or higher stability against proteolytic degradation of the light chain and to methods for the manufacture of such recombinant botulinum neurotoxins. These novel recombinant botulinum neurotoxins comprise a modification of the light chain of the neurotoxin, and the methods comprise the steps of inserting mutations at the light chain in the nucleic acid sequence coding for a parental botulinum neurotoxin and expression of the recombinant nucleic acid sequence comprising the mutated sequence in a host cell. The invention further relates to novel recombinant single-chain precursor botulinum neurotoxins used in such methods, nucleic acid sequences encoding such recombinant single-chain precursor botulinum neurotoxins, and pharmaceutical compositions comprising the recombinant botulinum neurotoxin with a higher oxidative resistance and/or higher stability against proteolytic degradation of the light chain.

## Description

### FIELD OF THE INVENTION

This invention relates to novel recombinant botulinum neurotoxins serotype A with a stabilized light chain and to methods for the manufacture of such recombinant botulinum neurotoxins. These novel recombinant botulinum neurotoxins comprise at least one amino acid modification, wherein said at least one amino acid modification is located at position 1 to 438 of the light chain. The invention further relates to pharmaceutical compositions comprising said recombinant neurotoxins. The invention further relates to novel recombinant single-chain precursor botulinum neurotoxins and nucleic acid sequences encoding such recombinant single-chain precursor botulinum neurotoxins.

### BACKGROUND OF THE INVENTION

Clostridium is a genus of anaerobe gram-positive bacteria, belonging to the Firmicutes. Clostridium consists of around 100 species that include common free-living bacteria as well as important pathogens, such as *Clostridium botulinum* and *Clostridium tetani.* Both species produce neurotoxins, botulinum toxin and tetanus toxin, respectively. These neurotoxins are potent inhibitors of calcium-dependent neurotransmitter secretion of neuronal cells and are among the strongest toxins known to man. The lethal dose in humans lies between 0.1 ng and 1 ng per kilogram of body weight.

Oral ingestion of botulinum toxin via contaminated food or generation of botulinum toxin in wounds can cause botulism, which is characterised by paralysis of various muscles. Paralysis of the breathing muscles can cause death of the affected individual.

Although both botulinum neurotoxin (BoNT) and tetanus neurotoxin (TxNT) function via a similar initial physiological mechanism of action, inhibiting neurotransmitter release from the axon of the affected neuron into the synapse, they differ in their clinical response. While the botulinum toxin acts at the neuromuscular junction and other cholinergic synapses in the peripheral nervous system, inhibiting the release of the neurotransmitter acetylcholine and thereby causing flaccid paralysis, the tetanus toxin acts mainly in the central nervous system, preventing the release of the inhibitory neurotransmitters GABA (gamma-aminobutyric acid) and glycine by degrading the protein synaptobrevin. The consequent overactivity in the muscles results in generalized contractions of the agonist and antagonist musculature, termed a tetanic spasm (rigid paralysis).

While the tetanus neurotoxin exists in one immunologically distinct type, the botulinum neurotoxins are known to occur in seven different immunogenic types, termed BoNT/A through BoNT/H. Most *Clostridium botulinum* strains produce one type of neurotoxin, but strains producing multiple toxins have also been described.

Botulinum and tetanus neurotoxins have highly homologous amino acid sequences and show a similar domain structure. Their biologically active form comprises two peptide chains, a light chain of about 50 kDa and a heavy chain of about 100 kDa, linked by a disulfide bond. A linker or loop region, whose length varies among different clostridial toxins, is located between the two cysteine residues forming the disulfide bond. This loop region is proteolytically cleaved by an unknown clostridial endoprotease to obtain the biologically active toxin.

The molecular mechanism of intoxication by TeNT and BoNT appears to be similar as well: entry into the target neuron is mediated by binding of the C-terminal part of the heavy chain to a specific cell surface receptor; the toxin is then taken up by receptor-mediated endocytosis. The low pH in the so formed endosome then triggers a conformational change in the clostridial toxin which allows it to embed itself in the endosomal membrane and to translocate through the endosomal membrane into the cytoplasm, where the disulfide bond joining the heavy and the light chain is reduced. The light chain can then selectively cleave so called SNARE-proteins, which are essential for different steps of neurotransmitter release into the synaptic cleft, e.g. recognition, docking and fusion of neurotransmitter-containing vesicles with the plasma membrane. TeNT, BoNT/B, BoNT/D, BoNT/F, and BoNT/G cause proteolytic cleavage of synaptobrevin or VAMP (vesicle-associated membrane protein), BoNT/A and BoNT/E cleave the plasma membrane-associated protein SNAP-25, and BoNT/C cleaves the integral plasma membrane protein syntaxin and SNAP-25.

In *Clostridium botulinum,* the botulinum toxin is formed as a protein complex comprising the neurotoxic component and non-toxic proteins. The accessory proteins embed the neurotoxic component thereby protecting it from degradation by digestive enzymes in the gastrointestinal tract. Thus, botulinum neurotoxins of most serotypes are orally toxic. Complexes with, for example, 450 kDa or with 900 kDa are obtainable from cultures of *Clostridium botulinum.*

In recent years, botulinum neurotoxins have been used as therapeutic agents in the treatment of dystonias and spasms. Preparations comprising botulinum toxin complexes are commercially available, e.g. from Ipsen Ltd (Dysport^{®}) or Allergan Inc. (Botox^{®}). A high purity neurotoxic component, free of any complexing proteins, is for example available from Merz Pharmaceuticals GmbH, Frankfurt (Xeomin^{®}).

Clostridial neurotoxins are usually injected into the affected muscle tissue, bringing the agent close to the neuromuscular end plate, i.e. close to the cellular receptor mediating its uptake into the nerve cell controlling said affected muscle. Various degrees of neurotoxin spread have been observed. The neurotoxin spread is thought to depend on the injected amount and the particular neurotoxin preparation. It can result in adverse side effects such as paralysis in nearby muscle tissue, which can largely be avoided by reducing the injected doses to the therapeutically relevant level. Overdosing can also trigger the immune system to generate neutralizing antibodies that inactivate the neurotoxin preventing it from relieving the involuntary muscle activity. Immunologic tolerance to botulinum toxin has been shown to correlate with cumulative doses.

At present, clostridial neurotoxins are still predominantly produced by fermentation processes using appropriate Clostridium strains. However, industrial production of clostridial neurotoxin from anaerobic Clostridium culture is a cumbersome and time-consuming process. Due to the high toxicity of the final product, the procedure must be performed under strict containment. During the fermentation process, the single-chain precursors are proteolytically cleaved by an unknown clostridial protease to obtain the biologically active di-chain clostridial neurotoxin. The degree of neurotoxin activation by proteolytic cleavage varies between different strains and neurotoxin serotypes, which is a major consideration for the manufacture due to the requirement of neurotoxin preparations with a well-defined biological activity. Furthermore, during fermentation processes using Clostridium strains the clostridial neurotoxins are produced as protein complexes, in which the neurotoxic component is embedded by accessory proteins. These accessory proteins have no beneficial effect on biological activity or duration of effect. They can however trigger an immune reaction in the patient, resulting in immunity against the clostridial neurotoxin. Manufacture of recombinant clostridial neurotoxins, which are not embedded by auxiliary proteins, might therefore be advantageous.

Methods for the recombinant expression of clostridial neurotoxins in *E. coli* are well known in the art (see, for example, WO 00/12728, WO 01/14570, or WO 2006/076902). Furthermore, clostridial neurotoxins have been expressed in eukaryotic expression systems, such as in *Pichia pastoris, Pichia methanolica, Saccharomyces cerevisiae,* insect cells and mammalian cells (see WO 2006/017749).

Recombinant botulinum neurotoxins may be expressed as single-chain precursors, which subsequently have to be proteolytically cleaved to obtain the final biologically active botulinum neurotoxin. Thus, botulinum neurotoxins may be expressed in high yield in rapidly-growing bacteria as relatively non-toxic single-chain polypeptides.

### OBJECTS OF THE INVENTION

It was an object of the invention to provide recombinant botulinum neurotoxins serotype A comprising a modified light chain with an increased stability against oxidative and/or thermo- degradation and/or a decreased vulnerability against proteolytic degradation. It was also an object of the invention to provide a pharmaceutical or cosmetic composition comprising recombinant botulinum neurotoxins serotype A with improved properties. It was a further object of the invention to establish a reliable and accurate method for manufacturing and obtaining such recombinant botulinum neurotoxins. Such a method and novel precursor botulinum neurotoxins used in such methods would serve to satisfy the great need for recombinant botulinum neurotoxins with a stabilized light chain.

### SUMMARY OF THE INVENTION

Botulinum neurotoxin type A can be inactivated by oxidation or by thermal stress or it can be cleaved by proteases. To make the protein more stable in vitro and in vivo methionine and cysteine residues of the enzymatic domain of the neurotoxin (the light chain) were mutated to oxidation resistant amino acids. Further to protect the light chain against proteolytic degradation loops exposed at the surface of the light chain were modified with the goal to make these loops tighter and less exposed.

Botulinum toxin can be inactivated during storage and in vivo by different mechanisms. Oxygen especially reactive oxygen species can attack sensitive residues and oxidize the protein which can lead to an inactive protein. Disufides can be formed by oxidation of cystein residues. Especially methionine residues are prone to become oxidized to methionine sulfoxide. To increase the stability of botulinum toxin sensitive methionine and cysteine were substituted by mutation with amino acids which are less sensitive to oxidation, e.g. leucine, isoleucine, lysine, glutamine or phenylalanine.

The vulnerability of cysteine and methionine residues was evaluated by estimating the solvent accessible surface area of the residues in the structure of the light chain structure by Molecular Dynamics simulation. It was postulated that the higher the solvent accessibility the higher the probability of the residue to become oxidized. The following residues were found to be highly solvent exposed and therefore sensible to oxidation: Met411, Met253, Met106, Met344, Cys134 and Met30 were found to be partially exposed whereas Met313 and Cys165 were analysed to be more buried in the structure of the light chain of botulinum neurotoxin type A.

By applying in silico design of mutations suitable amino acids were determined to substitute each of the identified methionine and cysteine residues in order to make the light chain and consequently the neurotoxin more stable which is listed in the following table, whereas single- point mutations are illustrated in single letter code.

**Table 1**

| **residue** | | **single-point mutations** |
|---|---|---|
| Met411 | | Y,F,V,K |
| Met253 | | L,V,Y,Q |
| Met106 | | I,H,L |
| Met344 | | L,I,T,V |
| Cys134 | | L,I,T |
| Met30 | | F,I,Q |
| Met313 | | L,I,Y |
| Cys165 | | L,I,T |

A protein can also be degraded by proteolytic attack. Proteases cleave proteins especially at surface loops which are exposed from the bulk of the protein and therefore accessible for proteolytic enzymes. Using the concept of thermostabilzation the following surface loops were identified and modified:

**Table 2 Loop regions**

| Region of the light chain of botulinum toxin type A | Residue range |
|---|---|
| Loop 20/30 | AS 25-29 |
| Loop 60/70 | AS 62-67 |
| Loop 120/130 | AS 121-126 |
| Loop 140 | AS 138-143 |
| ω loop 170/180 | AS169-173 |
| Loop 300 | AS310-309 |

In order to stabilize the light chain against thermal denaturation and against proteolytic attack I) amino acids in the loop were deleted ii) disulphide bridges were introduced by mutation iii) tryptophane bridges were introduced by mutation and/or loops were redesigned to make them tighter and less exposed.

Surprisingly, it could be found that a modified light chain of botulinum toxin type A with exchange of sensitive methionine and cysteine residues was less vulnerable against oxidation under harsh oxidative conditions. In addition, it was surprisingly found that mutated light chain molecules with modified loops were more resistant against proteases like trypsin or pronase.

So far, there is no generally applicable method for modifying botulinum neurotoxins in order to stabilize them against degradation. Surprisingly, it has been found that recombinant botulinum neurotoxins serotype A having such a property can be obtained by modifying amino acid residues of the light chain of botulinum neurotoxin serotype A, and by subsequent heterologous expression of the generated construct in recombinant host cells.

Thus, in one aspect, the present invention relates to recombinant botulinum neurotoxin serotype A comprising at least one amino acid modification, wherein said at least one amino acid modification is located in the sequence of the light chain (AS 1-438), wherein said at least one amino acid modification confers a more stable light stain than an otherwise identical botulinum neurotoxin lacking said at least one amino acid modification.

In another aspect, the present invention relates to a composition, in particular to a pharmaceutical composition comprising the recombinant botulinum neurotoxin of the present invention.

In yet another aspect, the present invention relates to the use of the composition of the present invention for cosmetic treatment.

In another aspect, the present invention relates to a method for the generation of the recombinant botulinum neurotoxin of the present invention, comprising the step of obtaining a recombinant nucleic acid sequence encoding a recombinant single-chain precursor botulinum neurotoxin by modifying the nucleic acid sequence at the light chain of the botulinum neurotoxin serotype A.

In another aspect, the present invention relates to a recombinant single-chain precursor botulinum neurotoxin comprising a modified light chain.

In another aspect, the present invention relates to a nucleic acid sequence encoding the recombinant single-chain precursor botulinum neurotoxin of the present invention.

In another aspect, the present invention relates to a method for obtaining the nucleic acid sequence of the present invention, comprising the step of modifying a nucleic acid sequence encoding the C-terminus of the light chain of the parental botulinum neurotoxin.

In another aspect, the present invention relates to a vector comprising the nucleic acid sequence of the present invention, or the nucleic acid sequence obtainable by the method of the present invention.

In another aspect, the present invention relates to a recombinant host cell comprising the nucleic acid sequence of the present invention, the nucleic acid sequence obtainable by the method of the present invention, or the vector of the present invention.

In another aspect, the present invention relates to a method for producing the recombinant single-chain precursor botulinum neurotoxin of the present invention, comprising the step of expressing the nucleic acid sequence of the present invention, or the nucleic acid sequence obtainable by the method of the present invention, or the vector of the present invention in a recombinant host cell, or cultivating the recombinant host cell of the present invention under conditions that result in the expression of said nucleic acid sequence.

### FIGURES

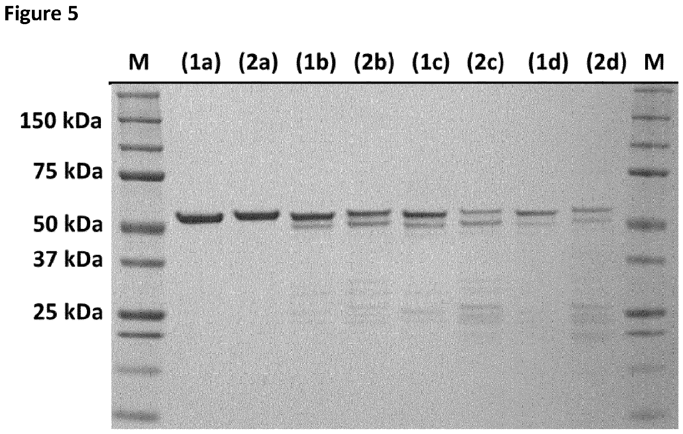

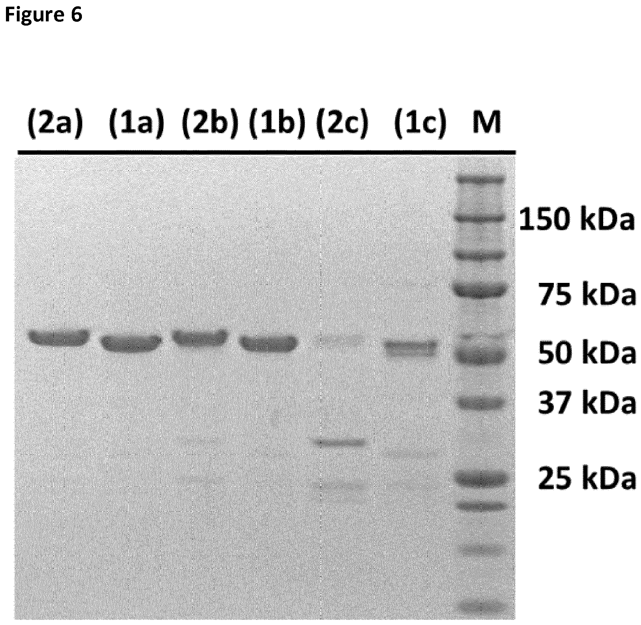

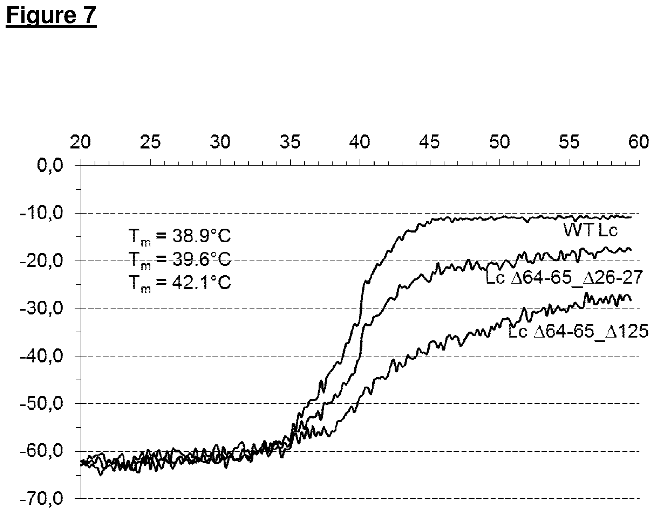

**Figure 1****: SDS-PAGE of the purified OxiSta6**
   All samples were reduced with mercaptoethanol before analysis. M Molecular weight marker, v.A OxiSta6 before activation with thrombin n.A. OxiSta6 after cleavage with thrombin, WT wildtype BoNT/A
   Sc single chain toxin
   Hc heavy chain
   Lc light chain
**Figure 2****: Endopeptidase -Activity after treatment with 50 mM H₂O₂** Mutants and Wildtyp of BoNT/A-Lc were treated with 50mM **H₂O₂** and the activity determined in the endopeptidaseassay (t0 = 100%)
   1 Wildtype BoNT/A
   2 DaSch018 (OxiSta4 - Met106Leu, Met253Leu, Met411 Gin)
   3 DaSch034 (OxiSta6 - Met106Leu, Met253Leu, Met 411Gln, Met30Phe, Cys134Ile, Cys165Ile, Met313Ile, Met344Leu)
   4 DaSch036 (OxiSta8 - Met106Leu, Met253Leu, Met411Gln, Met30Tyr, Cys134Ile, Met344Leu
**Figure 3****: SDS-PAGE of purified BoNT/A-Lc_Δ6465.**
   All samples were reduced with mercaptoethanol before analysis. M Molecular weight marker, v.I before Induction, n.I. after Induction. P cell pellet, L cell lysate, A4-A8 fractions of the affinity chromatography. The overexpressed protein can be seen at about 50kD corresponding with the Lc of BoNT/A1.

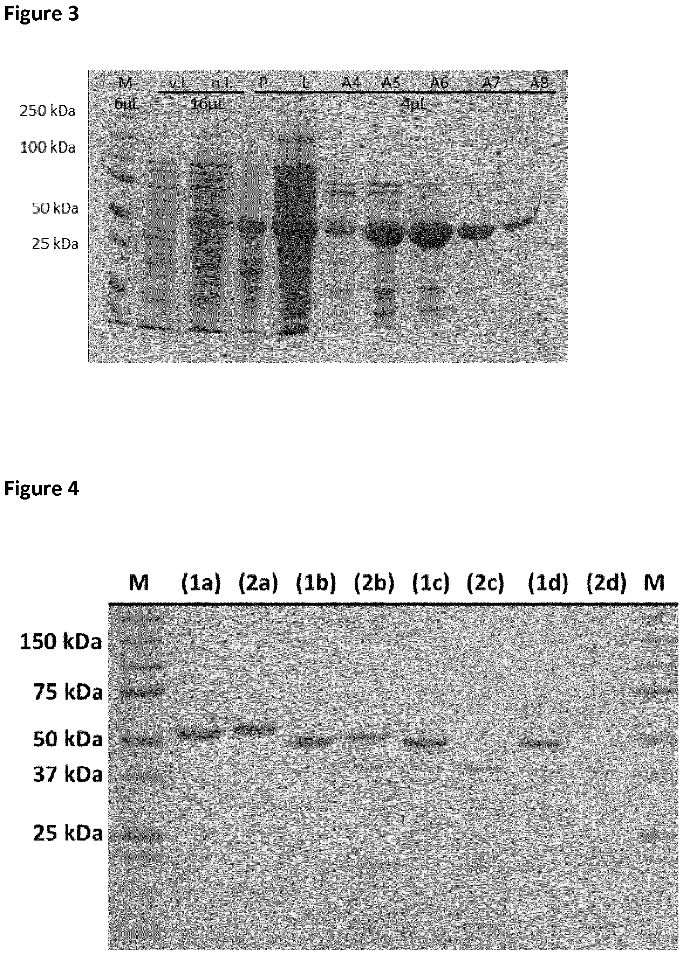

**Figure 4****: SDS-PAGE of BoNT/A_LC_Δ2627_Δ6465_Δ125 after Incubation with Trypsin**
   All samples were reduced with mercaptoethanol before analysis. M Molecular weight marker.
   (1) BoNT/A Lc_Δ26/27_Δ64/65_Δ125
   (2) Wildtype light chain
      a: after 1 hrs incubation with trypsin
      b: after 2 hrs incubation with trypsin
      c: after 8 hrs incubation with trypsin
**Figure 5****: SDS-PAGE of BoNT/A_LC_Δ2627_Δ6465_Δ125 after Incubation with Pronase**
   All samples were reduced with mercaptoethanol before analysis. M Molecular weight marker.
   (1) BoNT/A Lc-_Δ26/27_Δ64/65_Δ125
   (2) Wildtype light chain
      a: after 1 hrs incubation with pronase
      b: after 2 hrs incubation with pronase
      c: after 8 hrs incubation with pronase
**Figure 6** **SDS-PAGE of BoNT/A_LC_Δ2627_Δ6465_Δ125 after Incubation with µ-Calpain**
   All samples were reduced with mercaptoethanol before analysis. M Molecular weight marker.
   (1) BoNT/A Lc-_Δ26/27_Δ64/65-Δ125
   (2) Wildtype light chain
      a: after 1 hrs incubation with µ-Calpain
      b: after 2 hrs incubation with µ-Calpain
      c: after 8 hrs incubation with µ-Calpain
**Figure 7** **Assessment of thermal stability by CD measurement** BoNT/A-Lc_Δ2627_Δ6465, BoNT/A-Lc_Δ6465_Δ125 and WT-BoNT-Lc were measure by Circular Dichroism spectroscopy at 222 nm.

### DETAILED DESCRIPTION OF THE INVENTION

The present invention may be understood more readily by reference to the following detailed description of the invention and the examples included therein.

In one aspect, the present invention relates to a recombinant botulinum neurotoxin serotype A or a functional variant thereof comprising at least one amino acid modification, wherein said at least one amino acid modification is located in the sequence of the light chain (AS 1 to 438) with the amino acid sequence numbering as in GenBank accession number YP_001253342.1 or in SEQ ID NO: 1, wherein said at least one amino acid modification confers a higher thermostability and/or a higher oxidative resistance and/or higher stability against proteolytic degradation of the light chain than an otherwise identical botulinum toxin light chain lacking said at least one amino acid modification.

In the context of the present invention, the term "botulinum neurotoxin" refers to a natural neurotoxin obtainable from bacteria *Clostridium botulinum,* or to a neurotoxin obtainable from alternative sources, including from recombinant technologies or from genetic or chemical modification. Particularly, the botulinum neurotoxins have endopeptidase activity.

Botulinum neurotoxins are produced as single-chain precursors that are proteolytically cleaved by an unknown clostridial endoprotease within the loop region to obtain the biologically active disulfide-linked di-chain form of the neurotoxin, which comprises two chain elements, a functionally active light chain and a functionally active heavy chain, where one end of the light chain is linked to one end of the heavy chain not via a peptide bond, but via a disulfide bond.

In the context of the present invention, the term "botulinum neurotoxin light chain" refers to that part of a botulinum neurotoxin that comprises an endopeptidase activity responsible for cleaving one or more proteins that is/are part of the so-called SNARE-complex involved in the process resulting in the release of neurotransmitter into the synaptic cleft: In naturally occurring botulinum neurotoxins, the light chain has a molecular weight of approx. 50 kDa.

In the context of the present invention, the term light chain comprises to the amino acids AS1-AS438 of BoNT/A representing the enzymatic domain of botulinum neurotoxin type A.

In the context of the present invention, the term "botulinum neurotoxin heavy chain" refers to that part of a botulinum neurotoxin that is responsible for entry of the neurotoxin into the neuronal cell: In naturally occurring botulinum neurotoxins, the heavy chain has a molecular weight of approx. 100 kDa.

In the context of the present invention, the term "functionally active botulinum neurotoxin chain" refers to a recombinant clostridial neurotoxin chain able to perform the biological functions of a naturally occurring *Clostridium botulinum* neurotoxin chain to at least about 50%, particularly to at least about 60%, to at least about 70%, to at least about 80%, and most particularly to at least about 90%, where the biological functions of botulinum neurotoxin chains include, but are not limited to, binding of the heavy chain to the neuronal cell, entry of the neurotoxin into a neuronal cell, release of the light chain from the di-chain neurotoxin, and endopeptidase activity of the light chain. Methods for determining a neurotoxic activity can be found, for example, in WO 95/32738, which describes the reconstitution of separately obtained light and heavy chains of tetanus toxin and botulinum toxin. Also cell-based assay methods as described for example in WO2009/114748, WO 2013/049508 and WO2014/207109.

In the context of the present invention, the term "about" or "approximately" means within 20%, alternatively within 10%, including within 5% of a given value or range. Alternatively, especially in biological systems, the term "about" means within about a log (i.e. an order of magnitude), including within a factor of two of a given value.

In the context of the present invention, the term "recombinant botulinum neurotoxin" refers to a composition comprising a botulinum neurotoxin that is obtained by expression of the neurotoxin in a heterologous cell such as *E. coli,* and including, but not limited to, the raw material obtained from a fermentation process (supernatant, composition after cell lysis), a fraction comprising a botulinum neurotoxin obtained from separating the ingredients of such a raw material in a purification process, an isolated and essentially pure protein, and a formulation for pharmaceutical and/or aesthetic use comprising a botulinum neurotoxin and additionally pharmaceutically acceptable solvents and/or excipients.

In the context of the present invention, the term "comprises" or "comprising" means "including, but not limited to". The term is intended to be open-ended, to specify the presence of any stated features, elements, integers, steps or components, but not to preclude the presence or addition of one or more other features, elements, integers, steps, components, or groups thereof. The term "comprising" thus includes the more restrictive terms "consisting of" and "consisting essentially of".

In a further aspect, the structural modification in the BoNT/A light chain comprises at least one amino acid modification in the light chain at at least one position selected from N26, A27, M30, E64, A65, M106, T125, C134, 1138, Q139, P140, D141, S143, Y144, C165, M253L, S302, I303, S309, L310, E325, M313, F331, M344 and M411.

In a further aspect, the structural modification in the BoNT/A light chain comprises at least two or three or four or five or six or seven or eight amino acid modifications.

In a further aspect, the structural modification in the BoNT/A light chain comprises one or more modifications selected from the group consisting of
(i) M30F modification (methionine at position AS30 of the light chain is replaced by a phenylalanine),
(ii) M30Y modification (methionine at position AS30 of the light chain is replaced by a tyrosine),
(iii) M106L modification (methionine at position AS106 is replaced by a leucine),
(iv) M106I modification (methionine at position AS106 is replaced by a isoleucine),
(v) C134I modification (cysteine at position AS134 is replaced by an isoleucine),
(vi) C165I modification (cysteine at position AS165 of the light chain is replaced by an isoleucine),
(vii) M253L modification (methionine at position AS253 of the light chain is replaced by a leucine),
(viii) M253V modification (methionine at position AS253 of the light chain is replaced by a valine),
(ix) M253Y modification (methionine at position AS253 of the light chain is replaced by a tyrosine),
(x) M3131 modification (methionine at position AS313 of the light chain is replaced by a isoleucine),
(xi) M344L modification (methionine at position AS344 of the light chain is replaced by an leucine),
(xii) M411 Q modification (methionine at position AS411 of the light chain is replaced by a glutamine),
(xiii) M411 F modification (methionine at position AS411 of the light chain is replaced by a phenylalanine),
(xiv) M411 K modification (methionine at position AS411 of the light chain is replaced by lysine),
(xv) Δ64 modification (deletion of a glutamic acid at position AS64),
(xvi) Δ65 modification (deletion of an alanine at position AS65
(xvii) Δ6465 modification (deletion of a glutamic acid at position AS64 and an alanine at position AS65),
(xviii) Δ26 modification (deletion of a asparagine at position AS26),
(xix) Δ27 modification (deletion of an alanine at position AS27)
(xx) Δ2627 modification (deletion of a asparagine at position AS26 and alanine at position AS27)
(xxi) Δ122 modification (deletion of a threonine at position AS122)
(xxii) Δ125 modification (deletion of a threonine at position AS125)
(xxiii) Δ140 modification (deletion of a proline at position AS140)
(xxiv) Δ141 modification (deletion of an aspartic acid at position ΔS141)
(xxv) Y144W and I138W modification (tyrosine at position AS144 is replaced by a tryptophane and isoleucine at position AS138 is replaced by tryptophane)
(xxvi) E325W/F331W modification (glutamic acid at position AS325 is replaced by a tryptophane and phenlyalanine at position AS331 is replaced by tryptophane)
(xxvii) I303C/L310C modification (isoleucine at position AS303 is replaced by a cysteine an leucine at position AS310 is replaced by cysteine)
(xxviii) S302C/S309C modification (serine at position AS302 is replaced by a cysteine and serine at position AS309 is replaced by cysteine)
(xxix) I138W/Q139E/P140N/S143K/Y144W ΔD141/ modification (isoleucine at position AS138 is replaced by tryptophane, glutamine at position AS139 is replaced by glutamic acid, proline at position AS140 is replaced by glutamine, serine at position AS143 is replaced by lysine and tyrosine at position AS144 is replaced by tryptophane whereas aspartic acid at position AS141 is deleted).

In particular embodiments, the recombinant botulinum neurotoxin serotype A comprises three amino acid modifications, wherein these modifications are located in light chain at position M106, M253 and M411, in particular wherein these amino acids are substituted as follows M106I, M253L, M411 F as found in SEQ ID NO 2.

In particular embodiments, the recombinant botulinum neurotoxin serotype A comprises three amino acid modifications, wherein these modifications are located in light chain at position M106, M253 and M411, in particular wherein these amino acids are substituted as follows M106I, M253V, M411 F as found in SEQ ID NO 3.

In particular embodiments, the recombinant botulinum neurotoxin serotype A comprises three amino acid modifications, wherein these modifications are located in light chain at position M106, M253 and M411, in particular wherein these amino acids are substituted as follows M106I, M253V, M411 K as found in SEQ ID NO 4.

In particular embodiments, the recombinant botulinum neurotoxin serotype A comprises three amino acid modifications, wherein these modifications are located in light chain at position M106, M253 and M411, in particular wherein these amino acids are substituted as follows M106L, M253L, M411 Q as found in SEQ ID NO 5.

In particular embodiments, the recombinant botulinum neurotoxin serotype A comprises three amino acid modifications, wherein these modifications are located in light chain at position M106, M253 and M411, in particular wherein these amino acids are substituted as follows M106L, M253Y, M411 K as found in SEQ ID NO 6.

In particular embodiments, the recombinant botulinum neurotoxin serotype A comprises eight amino acid modifications, wherein these modifications are located in light chain at position M30, M106, C134, C165, M253, M313, M344, M411, in particular wherein these amino acids are substituted as follows M30F, M106L C134I C165I, M253L, M313I, M344L, M411Q as found in SEQ ID NO 7.

In particular embodiments, the recombinant botulinum neurotoxin serotype A comprises eight amino acid modifications, wherein these modifications are located in light chain at position M30, M106, C134, C165, M253, M313, M344, M411, in particular wherein these amino acids are substituted as follows M30F, M106L C134I C165I, M253Y, M313I, M344L, M411 K.

In particular embodiments, the recombinant botulinum neurotoxin serotype A comprises six amino acid modifications, wherein these modifications are located in light chain at position M30, M106, C134, M253, M344, M411, in particular wherein these amino acids are substituted as follows M30Y, M106L, C134I, M253L, M344L, M411Q as found in SEQ ID NO 8.

In particular embodiments, the recombinant botulinum neurotoxin serotype A comprises a deletion of N26 and A27 as found in SEQ ID NO 9.

In particular embodiments, the recombinant botulinum neurotoxin serotype A comprises a deletion of E64 and A65 as found in SEQ ID NO 10.

In particular embodiments, the recombinant botulinum neurotoxin serotype A comprises a deletion of T125 as found in SEQ ID NO 11.

In particular embodiments, the recombinant botulinum neurotoxin serotype A comprises a deletion of T122 as found in SEQ ID NO 12.

In particular embodiments, the recombinant botulinum neurotoxin serotype A comprises a deletion of E64 and A65 and a deletion of T125 as found in SEQ ID NO 13.

In particular embodiments, the recombinant botulinum neurotoxin serotype A comprises a deletion of N26 and A27, a deletion of E64 and A65 and a deletion of T125 as found in SEQ ID NO 14.

In particular embodiments, the recombinant botulinum neurotoxin serotype A comprises a deletion of E64 and A65 and a deletion of T122 as found in SEQ ID NO 15.

In particular embodiments, the recombinant botulinum neurotoxin serotype A comprises a deletion of N26 and A27 and a deletion of T122 as found in SEQ ID NO 16.

In particular embodiments, the recombinant botulinum neurotoxin serotype A comprises a deletion of E64 and A65 and a replacement of 1138 and Y144 by tryptophane as found in SEQ ID NO 17.

In particular embodiments, the recombinant botulinum neurotoxin serotype A comprises a replacement of E325 and Y331 by tryptophane as found in SEQ NO18.

In particular embodiments, the recombinant botulinum neurotoxin serotype A comprises a deletion of E64 and A65 and a replacement of I303 and L310 by cysteine as found in SEQ ID NO19.

In particular embodiments, the recombinant botulinum neurotoxin serotype A comprises a deletion of P140 and a replacement of 1138 and Y144 by tryptophane and Q139 by glutamic acid D141 by asparagine and S143 by lysine as found in SEQ ID NO 20.

In particular embodiments, the recombinant botulinum neurotoxin serotype A comprises a deletion of E64 and A65 and a replacement of 1138 and Y144 by tryptophane, a deletion of P140 and a replacement of Q139 by glutamic acid and D141 by asparagine and S143 by lysine as found in SEQ ID NO 21.

In particular embodiments, the recombinant botulinum neurotoxin serotype A comprises a deletion of E64 and A65 and a replacement of Q139 by glutamic acid, a deletion of P140 and D141 by asparagine and S143 by lysine as found in SEQ ID NO 22.

In a further aspect, the structural modification in the BoNT/A light chain comprises at least one amino acid modification in the light chain at least one deletion of N26, A27, E64, A65, T122, T125 or D141 and an introduction of a Trp/Trp pair by replacing Y144 and 1138 or E325 and F331 by tryptophane and formation of a disulphide bridge by replacing S302 and S309 or I303 and L310 by cysteine and by redesigning loop 140 by exchanging of Q139, P140 and S143 by glutamic acid glutamine and lysine, respectively.

In a further aspect the recombinant botulinum neurotoxin serotype A comprises amino acid modifications, wherein amino acid modifications are located in the light chain at position 1138, Y144, I303, L310, E325, F331, S302C and S309C, wherein these amino acids are pairwise substituted as follows I138W and Y144W, E325W and F331W, S302C and S309C, I303C and L310C.

In a further aspect, the recombinant botulinum neurotoxin serotype A comprises amino acid modifications, wherein amino acid modifications are located in the light chain at position 1138 Q139, P140, D141 S143 and Y144, wherein D141 is deleted and the other amino acids are substituted as follows I38W, Q139E, P140N, S143K and Y144W.

In the context of the present invention, the term "functional variant of a botulinum neurotoxin" refers to a neurotoxin that differs in the amino acid sequence and/or the nucleic acid sequence encoding the amino acid sequence from a botulinum neurotoxin, but is still functionally active. In the context of the present invention, the term "functionally active" refers to the property of a recombinant botulinum neurotoxin to exhibit a biological activity of at least about 20 %, particularly to at least about 40%, at least about 70%, at least about 80%, and most particularly at least about 90% of the biological activity of a naturally occurring parental botulinum neurotoxin, i.e. a parental botulinum neurotoxin without modifications at the C-terminus of the light chain, where the biological functions include, but are not limited to, binding to the neurotoxin receptor, entry of the neurotoxin into a neuronal cell, release of the light chain from the two-chain neurotoxin, and endopeptidase activity of the light chain, and thus inhibition of neurotransmitter release from the affected nerve cell. In vivo assays for assessing biological activity include the mouse LD50 assay and the ex vivo mouse hemidiaphragm assay as described by Pearce et al. (Pearce 1994, Toxicol. Appl. Pharmacol. 128: 69-77) and Dressler et al. (Dressler 2005, Mov. Disord. 20:1617-1619, Keller 2006, Neuroscience 139: 629-637) or a cell-based assay as described in WO2009/114748, WO2014/207109 or WO 2013/049508. The biological activity is commonly expressed in Mouse Units (MU). As used herein, 1 MU is the amount of neurotoxic component, which kills 50% of a specified mouse population after intraperitoneal injection, i.e. the mouse i.p. LD50.

On the protein level, a functional variant will maintain key features of the corresponding botulinum neurotoxin serotype A, such as key residues for the endopeptidase activity in the light chain, or key residues for the attachment to the neurotoxin receptors or for translocation through the endosomal membrane in the heavy chain, but may contain modifications comprising a substitution of one or more amino acids of the corresponding botulinum neurotoxin.

In another embodiment, the functional variant of a botulinum neurotoxin additionally comprises a signal peptide. Usually, said signal peptide will be located at the N-terminus of the neurotoxin. Many such signal peptides are known in the art and are comprised by the present invention. In particular, the signal peptide results in transport of the neurotoxin across a biological membrane, such as the membrane of the endoplasmic reticulum, the Golgi membrane or the plasma membrane of a eukaryotic or prokaryotic cell. It has been found that signal peptides, when attached to the neurotoxin, will mediate secretion of the neurotoxin into the supernatant of the cells. In certain embodiments, the signal peptide will be cleaved off in the course of, or subsequent to, secretion, so that the secreted protein lacks the N-terminal signal peptide, is composed of separate light and heavy chains, which are covalently linked by disulfide bridges, and is proteolytically active.

In particular embodiments, the functional variant has a sequence identity of at least about 40%, at least about 50%, at least about 60%, at least about 70% or most particularly at least about 80%, and a sequence homology of at least about 60%, at least about 70%, at least about 80%, at least about 90%, or most particularly at least about 95% to the corresponding part in the parental botulinum neurotoxin serotype A. Methods and algorithms for determining sequence identity and/or homology, including the comparison of variants having deletions, additions, and/or substitutions relative to a parental sequence, are well known to the practitioner of ordinary skill in the art. The term "identity" as used herein refers to sequence identity characterized by determining the number of identical amino acids between two nucleic acid sequences or two amino acid sequences wherein the sequences are aligned so that the highest order match is obtained. It can be calculated using published techniques or methods codified in computer programs such as, for example, BLASTP, BLASTN or FASTA (Altschul 1990, J Mol Biol 215, 403). The percent identity values are, in one aspect, calculated over the entire amino acid sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS 5, 151) or the programs Gap and BestFit (Needleman 1970, J Mol Biol 48; 443; Smith 1981, Adv Appl Math 2, 482), which are part of the GCG software packet (Genetics Computer Group 1991, 575 Science Drive, Madison, Wisconsin, USA 53711), may be used. The sequence identity values recited above in percent (%) are to be determined, in another aspect of the invention, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments. On the DNA level, the nucleic acid sequences encoding the functional homologue and the parental botulinum neurotoxin may differ to a larger extent due to the degeneracy of the genetic code. It is known that the usage of codons is different between prokaryotic and eukaryotic organisms. Thus, when expressing a prokaryotic protein such as a botulinum neurotoxin, in a eukaryotic expression system, it may be necessary, or at least helpful, to adapt the nucleic acid sequence to the codon usage of the expression host cell, meaning that sequence identity or homology may be rather low on the nucleic acid level.

In the context of the present invention, the term "variant" refers to a botulinum neurotoxin that is a chemically, enzymatically, or genetically modified derivative of a corresponding neurotoxin of *C. botulinum* neurotoxin serotype A. A chemically modified derivative may be one that is modified by pyruvation, phosphorylation, sulfatation, lipidation, pegylation, glycosylation and/or the chemical addition of an amino acid or a polypeptide comprising between 2 and about 100 amino acids, including modification occurring in the eukaryotic host cell used for expressing the derivative. An enzymatically modified derivative is one that is modified by the activity of enzymes, such as endo- or exoproteolytic enzymes, including modification by enzymes of the eukaryotic host cell used for expressing the derivative. As pointed out above, a genetically modified derivative is one that has been modified by deletion or substitution of one or more amino acids contained in, or by addition of one or more amino acids (including polypeptides comprising between 2 and about 100 amino acids) to, the amino acid sequence of said botulinum neurotoxin. Methods for designing and constructing such chemically or genetically modified derivatives and for testing of such variants for functionality are well known to anyone of ordinary skill in the art.

In particular embodiments, said recombinant botulinum neurotoxin serotype A comprising at least one amino acid modification, wherein said at least one amino acid modification is located in the light chain of Clostridium botulinum toxin typeA at position 1 to 438 showing a higher thermostability and/or a higher oxidative resistance and/or higher stability against proteolytic degradation of the light chain than an identical botulinum neurotoxin without the modifications of the light chain.

In the context of the present invention the term "higher thermostability" refers to a mutant which is unfolded at a higher temperature (mid point of thermal transition) than the botulinum toxin without said mutations. The mid point of thermal transition can be assessed as described in example 10.

In the context of the present invention the term "higher oxidative resistance" refers to a mutant whose specific enzymatic activity after oxidative stress is more than 20%, particularly more than 50% and particularly more than 90% higher than the specific activity of the botulinum toxin without said mutations under oxidative stress. The oxidative resistance of botulinum toxin can be measured by determining the endopeptidase activity after treatment with H₂O₂ as described in example 5.

In the context of the present invention the term "higher stability against proteolytic degradation" refers to a mutant which shows 20% less degradation products, particularly less than 50%, more particularly less than 90% degradation (products) than the botulinum toxin without said mutation(s) when incubated with proteolytic enzymes. The proteolytic degradation of botulinum toxin can be measured by incubating the botulinum toxin with proteases like pronase, trypsin or calpain and analyzing the samples with SDS-PAGE and densitometric measurements as described in examples 6, 7, 8 and 9.

In the context of the present invention the term "denervation" refers to denervation resulting from administration of a chemodenervating agent, for example a neurotoxin.

In the context of the present invention, the term "localized denervation" or "localized paralysis" refers to denervation of a particular anatomical region, usually a muscle or a group of anatomically and/or physiologically related muscles, which results from administration of a chemodenervating agent, for example a neurotoxin, to the particular anatomical region.

In particular embodiments, the recombinant botulinum neurotoxin is for the use in the treatment of a disease requiring chemodenervation, wherein the recombinant botulinum neurotoxin is characterized by a higher chemical and biological stability

In another aspect, the present invention relates to a pharmaceutical or cosmetic composition comprising the recombinant botulinum neurotoxin of the present invention. For preparing a pharmaceutical preparation comprising a botulinum neurotoxin the toxin can be formulated by various techniques dependent on the desired application purposes which are known in the art. For example, the (biologically active) botulinum neurotoxin polypeptide can be used in combination with one or more pharmaceutically acceptable carriers as a pharmaceutical composition. The pharmaceutically acceptable carrier(s) must be acceptable in the sense of being compatible with the other ingredients of the formulation and being not deleterious to the recipient thereof. The pharmaceutical carrier employed may include a solid, a gel, or a liquid. Exemplary of solid carriers are lactose, terra alba, sucrose, talc, gelatine, agar, pectin, acacia, magnesium stearate, stearic acid and the like. Exemplary of liquid carriers are glycerol, phosphate buffered saline solution, water, emulsions, various types of wetting agents, and the like. Suitable carriers comprise those mentioned above and others well known in the art, see, e.g., Remington's Pharmaceutical Sciences, Mack Publishing Company, Easton, Pennsylvania. In an aspect, the pharmaceutical composition can be dissolved in a diluent, prior to administration. The diluent is also selected so as not to affect the biological activity of the Neurotoxin product. Examples of such diluents are distilled water or physiological saline. In addition, the pharmaceutical composition or formulation may also include other carriers or non-toxic, non-therapeutic, non-immunogenic stabilizers and the like. Thus, the formulated Neurotoxin product can be present, in an aspect, in liquid or lyophilized form. In an aspect, it can be present together with glycerol, protein stabilizers (HSA) or non-protein stabilizers such as polyvinyl pyrrolidone (PVP), hyaluronic acid or free amino acids. In an aspect, suitable non-proteinaceous stabilizers are disclosed in WO 2005/007185 or WO 2006/020208. The formulated Neurotoxin product may be used for human or animal therapy of various diseases or disorders in a therapeutically effective dose or for cosmetic purposes.

In particular embodiments, the recombinant botulinum neurotoxin of the present invention or the pharmaceutical composition of the present invention is for use in the treatment of a disease or condition taken from the list of:
wound healing, immobilisation for bone and tendon fracture treatment, post surgery immobilization, specifically in connection with haemorrhoidectomy, introduction of dental implants, or hip joint replacement (endoprothesis), epicondylitis, knee arthroplasty, ophthalmological surgery, acne, irritable bowel disease, vaginism, low back pain, or benign prostate dysplasia.

In yet another aspect, the present invention relates to the use of the composition of the present invention for cosmetic treatment.

In the context of the present invention, the term "cosmetic treatment" relates to uses in cosmetic or aesthetic applications, such as the treatment of wrinkles, crow's feet, glabella frown lines, reduction of the masseter muscle, reduction of the calves, removing of facial asymmetries etc..

In another aspect, the present invention relates to a method for the generation of the recombinant botulinum neurotoxin of the present invention, comprising the step of obtaining a recombinant nucleic acid sequence encoding a recombinant single-chain precursor botulinum neurotoxin by the modifying the nucleic acid sequence encoding a parental botulinum neurotoxin.

In the context of the present invention, the term "recombinant nucleic acid sequence" refers to a nucleic acid, which has been generated by joining genetic material from two different sources.

In the context of the present invention, the term "single-chain precursor botulinum neurotoxin" refers to a single-chain precursor for a disulfide-linked di-chain botulinum neurotoxin, comprising a functionally active botulinum neurotoxin light chain, a functionally active neurotoxin heavy chain, and a loop region linking the C-terminus of the light chain with the N-terminus of the heavy chain.
In the context of the present invention, the term "recombinant single-chain precursor botulinum neurotoxin" refers to a single-chain precursor botulinum neurotoxin comprising a modified light chain of the neurotoxin.

In particular embodiments, the recombinant single-chain precursor botulinum neurotoxin comprises a protease cleavage site in said loop region.

Single-chain precursor botulinum neurotoxins have to be proteolytically cleaved to obtain the final biologically active botulinum neurotoxins. Proteolytic cleavage may either occur during heterologous expression by host cell enzymes, or by adding proteolytic enzymes to the raw protein material isolated after heterologous expression. Naturally occurring botulinum neurotoxins usually contain one or more cleavage signals for proteases which post-translationally cleave the single-chain precursor molecule, so that the final di- or multimeric complex can form. At present, botulinum neurotoxins are still predominantly produced by fermentation processes using appropriate Clostridium strains. During the fermentation process, the single-chain precursors are proteolytically cleaved by an unknown clostridial protease to obtain the biologically active di-chain clostridial neurotoxin. In cases, where the single-chain precursor molecule is the precursor of a protease, autocatalytic cleavage may occur. Alternatively, the protease can be a separate non-clostridial enzyme expressed in the same cell. WO 2006/076902 describes the proteolytic cleavage of a recombinant clostridial neurotoxin single-chain precursor at a heterologous recognition and cleavage site by incubation of the *E. coli* host cell lysate. The proteolytic cleavage is carried out by an unknown *E. coli* protease. In certain applications of recombinant expression, modified protease cleavage sites have been introduced recombinantly into the interchain region between the light and heavy chain of clostridial toxins, e.g. protease cleavage sites for human thrombin or non-human proteases (see WO 01/14570).

In particular embodiments, the protease cleavage site is a site that is cleaved by a protease selected from the list of: thrombin, trypsin, enterokinase, factor Xa, plant papain, insect papain, crustacean papain, enterokinase, human rhinovirus 3C protease, human enterovirus 3C protease, tobacco etch virus protease, Tobacco Vein Mottling Virus, subtilisin and caspase 3.

In a particular embodiment, the recombinant single-chain precursor botulinum neurotoxin further comprises a binding tag, particularly selected from the group comprising: glutathione-S-transferase (GST), maltose binding protein (MBP), a His-tag, a StrepTag, or a FLAG-tag.

In the context of the present invention, the term "parental botulinum neurotoxin" refers to an initial botulinum neurotoxin without modifications of the light chain, selected from a natural botulinum neurotoxin, a functional variant of a natural botulinum neurotoxin or a chimeric botulinum neurotoxin.

In particular embodiments, the method for the generation of the recombinant botulinum neurotoxin of the present invention further comprises the step of heterologously expressing said recombinant nucleic acid sequence in a host cell, particularly in a bacterial host cell, more particularly in an *E. coli* host cell.

In certain embodiments, the *E. coli* cells are selected from *E*. *coli* XL1-Blue, Nova Blue, TOP10, XL10-Gold, BL21, and K12.

In particular embodiments, the method for the generation of the recombinant botulinum neurotoxin of the present invention additionally comprises at least one of the steps of (i) generating a disulfide-linked di-chain recombinant botulinum neurotoxin comprising a modified C- terminus of the light chain by causing or allowing contacting of said recombinant single-chain precursor botulinum neurotoxin with an endoprotease and (ii) purification of said recombinant single-chain precursor botulinum neurotoxin or said disulfide-linked di-chain recombinant botulinum neurotoxin by chromatography.

In particular embodiments, the recombinant single-chain precursor botulinum neurotoxin, or the recombinant disulfide-linked di-chain botulinum neurotoxin, is purified after expression, or in the case of the recombinant disulfide-linked di-chain botulinum neurotoxin, after the cleavage reaction. In particular such embodiments, the protein is purified by chromatography, particularly by immunoaffinity chromatography, or by chromatography on an ion exchange matrix, a hydrophobic interaction matrix, or a multimodal chromatography matrix, particularly a strong ion exchange matrix, more particularly a strong cation exchange matrix.

In the context of the present invention, the term "causing ... contacting of said recombinant single-chain precursor botulinum neurotoxin ...with an endoprotease" refers to an active and/or direct step of bringing said neurotoxin and said endoprotease in contact, whereas the term "allowing contacting of a recombinant single-chain precursor botulinum neurotoxin ...with an endoprotease" refers to an indirect step of establishing conditions in such a way that said neurotoxin and said endoprotease are getting in contact to each other.

In the context of the present invention, the term "endoprotease" refers to a protease that breaks peptide bonds of non-terminal amino acids (i.e. within the polypeptide chain). As they do not attack terminal amino acids, endoproteases cannot break down peptides into monomers.

In particular embodiments, cleavage of the recombinant single-chain precursor botulinum neurotoxin is near-complete.

In the context of the present invention, the term "near-complete" is defined as more than about 95% cleavage, particularly more than about 97.5%, more particularly more than about 99% as determined by SDS-PAGE and subsequent Western Blot or reversed phase chromatography.

In particular embodiments, cleavage of the recombinant single-chain precursor botulinum neurotoxin occurs at a heterologous cleavage signal located in the loop region of the recombinant precursor botulinum neurotoxin.

In particular embodiments, the cleavage reaction is performed with crude host cell lysates containing said single-chain precursor protein.

In other particular embodiments, the single-chain precursor protein is purified or partially purified, particularly by a first chromatographic enrichment step, prior to the cleavage reaction.

In the context of the present invention, the term "purified" relates to more than about 90% purity. In the context of the present invention, the term "partially purified" relates to purity of less than about 90% and an enrichment of more than about two fold.

In another aspect, the present invention relates to a recombinant single-chain botulinum neurotoxin, which is a precursor for the recombinant botulinum neurotoxin of the present invention Thus, in such aspect, the present invention relates to a recombinant single-chain precursor botulinum neurotoxin comprising a modified light chain.

In another aspect, the present invention relates to a method for obtaining the nucleic acid sequence of the present invention, comprising the step of modifying a nucleic acid sequence encoding a parental botulinum neurotoxin.

In another aspect, the present invention relates to a vector comprising the nucleic acid sequence of the present invention, or the nucleic acid sequence obtainable by the method of the present invention.

In another aspect, the present invention relates to a recombinant host cell comprising the nucleic acid sequence of the present invention, the nucleic acid sequence obtainable by the method of the present invention, or the vector of the present invention.

In certain embodiments, the recombinant host cells are selected from *E. coli* XL1-Blue, Nova Blue, TOP10, XL10-Go!d, BL21, and K12.

In another aspect, the present invention relates to a method for producing the recombinant single-chain precursor botulinum neurotoxin of the present invention, comprising the step of expressing the nucleic acid sequence of the present invention, or the nucleic acid sequence obtainable by the method of the present invention, or the vector of the present invention in a recombinant host cell, or cultivating the recombinant host cell of the present invention under conditions that result in the expression of said nucleic acid sequence.

### EXAMPLES

### Example 1: Generation and Purification of a BoNT/A1 mutants with modifications of methionine and cysteine (OxiSta4-rBoNT/A1, OxiSta6 and OxiSta8)

### Molecular biology

For expression of BoNT/A in *E.coli* codon optimized gene constructs were cloned into pET29c (dna and protein sequence table TS1). These variants contain C-terminal fused His6- and Strep-affinity tags which can be cleaved after protein purification *via* thrombin. In order to construct a plasmid for the expression of BonT/A a synthetic gene (Thermo Scientific) with the corresponding mutations OxiSta3: Met106Ile, Met253Leu, Met411 Lys, OxiSta6: Met106Leu, Met253Leu, Met 411 Gin, Met30Phe, Cys134Ile, Cys165Ile, Met313Ile, Met344Leu or OxiSta8: Met106Leu, Met253Leu, Met 411 Gin, Met30Phe, Cys134Ile, Cys165Ile, Met313Ile, Met344Leu of the BoNT/A1 wild type sequence (SEQ ID NO 1) and restriction sites Sbfl and Seal was used.

### Protein expression and purification.

Expression of rBoNT/A variants was performed in Riesenberg media with 50 µg/mL Kanamycin{Riesenberg, 1991 #1}. Cells were grown in shake flasks (37°C, 175 r.p.m) until an OD600 of 1.5-2 was reached. For induction of protein expression 1 mM IPTG (Fermentas) was added to the E.coli culture. Protein synthesis was performed for 24 h (15 °C, 175 r.p.m.). Cells were collected by centrifugation (5,000 r.p.m., 20 min, 4 °C) and resuspended in His binding buffer pH 8.0 (50 mM Tris, 150 mM NaCl, 5 mM Imidazol) containing EDTA-free protease inhibitor complete (Roche Diagnostics). For the determination of endopeptidase activity and *in vivo* characterization, the different toxin variants were extracted and purified. Resuspended pellets were disrupted in 2-3 cycles by a French Press Cell Disrupter (Thermo Electron Corporation) at 4 °C. The resulting crude extracts were centrifuged (20,000 r.p.m., 30 min, 4 °C), and the supernatants with the soluble proteins were recovered. Protein purification was carried out by fast protein liquid chromatography (GE Healthcare) using a three step purification protocols. The first capture step was performed by IMAC using a HisTrap HP 1 mL column (GE Healthcare). The column was washed (1 ml min-1 working flow) using a two-step protocol with His elution buffer (50 mM Tris, 150 mM NaCl, 400 mM Imidazol pH 8,0). The elution of the toxin proteins occurred at 400 mM Imidazol. In a further step a Strep-Tactin affinity chromatography was performed as previously described (IBA GmbH). As an alternative instead of a second affinity chromatography a cation exchange chromatography with a HiTrap SP HP 1 mL column (GE Healthcare, Freiburg, Germany) was used. The corresponding samples were diluted with SP binding buffer (50 mM Tris, pH 8) and eluted with SP elution buffer (50 mM Tris, 1 M NaCl, pH 8). This procedure was followed by a SEC using a Superdex 200 10/300 column (GE Healthcare). The SEC running buffer (20 mM Tris, 150 mM NaCl, 2,5 mM CaCl2 pH 7,7) was also used to store the purified protein solutions in aliquots at -20 °C. Each protein was analyzed by applying 0,5-1 µg on 4-12% gradient SDS-PAGE (Novex Life Technologies) and stained with Coomassie G-250 based SimplyBlue safe stain (Pierce). Each protein was judged >98% pure before applying *in vitro* or *in vivo* experiments.

### Thrombin cleavage

BoNT/A preparations were activated with Thrombin (Merck Millipore; 8 U/1 mg BoNT) for 24 h at 20 °C yielding > 99% of di-chain toxin. Afterwards the cleavage protease was eliminated with the previously described Strep-Tactin Kit (IBA GmbH).

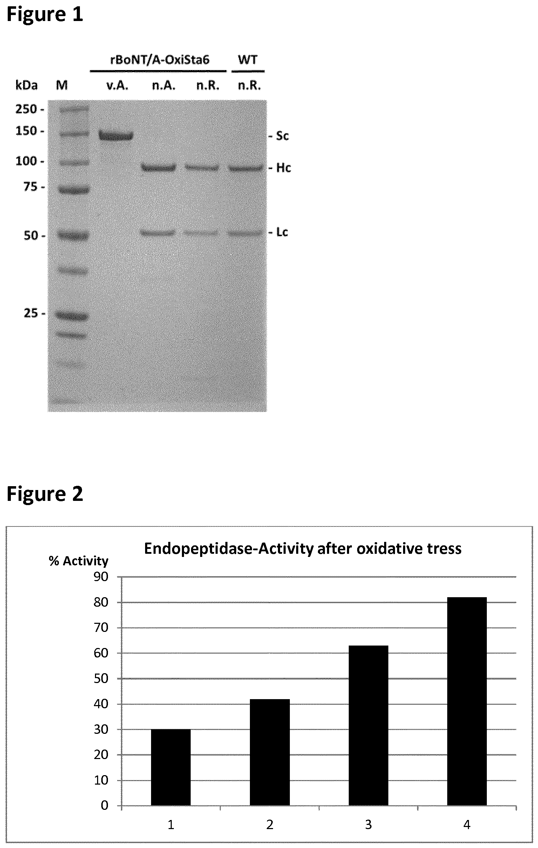

Purification was done using a combination of his affinity, ion exchange and size exclusion chromatography, followed by activation using thrombin. Figure 1 summarizes the results of purification and activation of rBoNT/A_OxiSta6: (Met106Leu, Met253Leu, Met 411 Gin, Met30Phe, Cys134Ile, Cys165Ile, Met313Ile, Met344Leu) as analyzed in SDS-PAGE.

### Example 2: Generation and Purification of a BoNT/A1 light chain mutants with modifications deletions, introduction of Trp/Trp pairs or disulphide bridges

### Molecular biology

For expression of BoNT/A in *E.coli* codon optimized gene constructs of the light chain (AS1-AS429) were cloned into pET29c. These variants contain C-terminal fused His6- and Strep-affinity tags which can be cleaved after protein purification *via* thrombin. The following mutation were introduced by site directed mutagenesis according to a protocol of Agilent (QucikChange II XL):
BoNT/A-Lc_Δ6465: Botulinum toxin type A light chain with deletion of A65 and E64;
BoNT/A-Lc_Δ2627: Botulinum toxin type A light chain with deletion of N26 and A27;
BoNT/A-Lc_Δ2627_Δ6465: Botulinum toxin type A light chain with deletion of N26, A27 A65 and E64;
BoNT/A-Lc_Δ6465_Δ125: Botulinum toxin type A light chain with deletion of A65, E64 and T125;
BoNT/A-Lc_Δ6465_I138W/Y144W: Botulinum toxin type A light chain with deletion of A65, E64 and a replacement of I138 and FY144 with tryptophane;
BoNT/A-Lc_Δ6465_E325W/F331W: Botulinum toxin type A light chain with deletion of A65, E64 and a replacement of E325 and F331 with tryptophane;
BoNT/A-LC_Δ6465_Δ2627_Δ125: Botulinum toxin type A light chain with deletion of N26, A27, E64, A65 and T125;
BoNT-Lc_Δ6465_I303C/L310C: Botulinum toxin type A light chain with deletion of A65, E64 and a replacement of I303 by cysteine and replacement of L310 by cysteine;
BoNT-Lc_I138W/Y144W: Botulinum toxin type A light chain with a replacement of 1138 and a replacement of Y144 by tryptophane;
BoNT-Lc_E325W/F331W: Botulinum toxin type A light chain with a replacement of I325 and a replacement of F331 by tryptophane;
BoNT-Lc_Δ6465_I302C/L310C: Botulinum toxin type A light chain with deletion of E64, A65 and a replacement of I303 by cysteine and replacement of L310 by cysteine;
BoNTLc_I138W_Q139E_Δ140_D141N/S143K_Y144W: Botulinum toxin type A light chain with a replacement of I38 and a replacement of Y144 by tryptophane a deletion of P140 and a replacement of D141 with asparagine and S143 with lysine;
BoNT-Lc_Q139E_A140_D141N/S143K: Botulinum toxin type A ligh chain with a replacement of Q139 with glutamic acid a deletion of P140 and a replacement of D141 with asparagine and S143 with lysine;
BoNT-Lc_Δ6465_I138W_Q139E_Δ140_D141N/S143K_Y144W: Botulinum toxin type A light chain with deletion of E64, A65 and a replacement of I38 and a replacement of Y144 by tryptophane and with a replacement of Q139 with glutamic acid a deletion of P140 and a replacement of D141 with asparagine and S143 with lysine;
BoNT-Lc_/Δ6465_Q139E_Δ140_D141N/S143K: Botulinum toxin type A light chain with deletion of E64, A65 and with a replacement of Q139 with glutamic acid, a deletion of P140 and a replacement of D141 with asparagine and S143 with lysine.

Restriction sites and restriction sites Sbfl and Scal were used.

### Protein expression and purification

Expression of LC/A variants was performed in Overnight Express™ Instant TB Medium with 50 µg/mL Kanamycin. Cells were grown in shake flasks (37°C, 175 r.p.m) until an OD600 of 0.4-0.7 was reached. For induction of protein expression the E.coli culture the temperature was decreases up to 25 °C, 130 r.p.m. Protein synthesis was performed for 24 h. Cells were collected by centrifugation (5,000 r.p.m., 20 min, 4 °C) and resuspended in His binding buffer pH 8.0 (50 mM Tris, 150 mM NaCl, 5 mM Imidazol) containing EDTA-free protease inhibitor complete (Roche Diagnostic). Resuspended pellets were disrupted in 2-3 cycles by a French Press Cell Disrupter (Thermo Electron Corporation) at 4 °C. The resulting crude extracts were centrifuged (20,000 r.p.m., 30 min, 4 °C), and the supernatants with the soluble proteins were recovered. Protein purification was carried out by fast protein liquid chromatography (GE Healthcare) using a two step purification protocol. The capture step was performed by IMAC using a HisTrap HP 1 mL column (GE Healthcare). The column was washed (1 ml min-1 working flow) using a two-step protocol with His elution buffer (50 mM Tris, 150 mM NaCl, 400 mM Imidazol pH 8,0). The elution of the toxin proteins occurred at 400 mM Imidazol. In a second step a gel filtration was performed using a 5 mL HiTrap Deslating column (GE Healthcare). The running buffer (5m mM phosphate buffer, pH 7,5) was also used to store the purified protein solutions in aliquots at -20 °C. Each protein was analyzed by applying 0,5-1 µg on 4-12% gradient SDS-PAGE (Novex Life Technologies) and stained with Coomassie G-250 based SimplyBlue safe stain (Pierce). Each protein was judged >90% pure before applying in vitro experiments.

Figure 3 shows an example for the purification of BoNT/A-Lc_Δ6465.

### Example 3: Determination of the enzymatic activity of light chain (endopeptidase assay)

Suitable concentrations of the mutants were reduced with 20mM TCEP (tris(2-carboxyethyl)phosphine reduced and then alkylated with 20 mM lodacetamide. For the testing the neurotoxin mutants were prepared in concentrations beween 0,0075U/mL and 0,140U/mL in a reaction buffer consisting of 0.1%Bovine serum albumin, 0.5%CHAPS, 20µM ZnCl₂ in 50 mM Hepes pH7.0. and then pipetted into microtiter plate well (MTP) which were incubated over night with the protease substrate 70ASS-HisSNAP25 (IBA, Gottingen). In the MTP a standard curve with Xeomin (Merz Pharmaceuticals GmbH) was analyzed in parall. After shaking for 120 min the reaction mixture was removed and after washing the MTP was incubated with an solution of an antibody which detect specifically the cleaved SNAP25 (anti-BoNT/A cleaved SNAP25-AK, R&D Antibodies). After 60 min incubation and washing a secondary antibody was added (anti-mouse-IgG (H&L HRP, Dianova). The concentration of bound antibody and thereby the endopeptidase activity was determined in the reaction with the peroxidase substrate tetramethylbenzidine (Perbio Science). The reaction was terminated by addition of 50µL 2M H2SO4 and the optical density at 450 measured. The activity of the sample was calculated in comparison with the standard which was used to create a standard curve.

### Example 4 Determination of the enzymatic activity of OxiStar4, OxiStar6 and OxiStar8

The enzymatic activity of the mutants was analyzed in the endopeptidase assay (Example 3) The results are shown in table 3

**Table 3**

| *in vitro* Characterization of the enzymatic activity in the endopeptidase-Assay of OxiSta4, OxiSta6 and OxiSta8 | |
|---|---|
| Variant | Spec. Activity [%] |
| Wildtype | 100* |
| OxiSta4 - (Met106Leu, Met253Leu, Met411Gln) | 90 - 100 |
| OxiSta6 -( Met106Leu, Met253Leu, Met 411Gln, Met30Phe, Cys134Ile, Cys165Ile, Met313Ile, Met344Leu) | 30-40 |
| OxiSta8-(Met106Leu, Met253Leu, Met411Gln, Met30Tyr, Cys134Ile, Met344Leu) | 30-40 |

### Example 5 Determination of the enzymatic activity of OxiStar4, OxiStar6 and OxiStar8 after incubation under oxidative condition (oxidative stress)

After reduction and alkylation (s. example 4) wildtype BoNT/A, OxiSta4, OxiSta6 and OxiSta8 were incubated with 50mM H2O2 in reaction buffer for 16 hrs. The samples were then analyzed for enzymatic activity as described in Example 4. The results are shown in figure 2. It was demonstrated that the mutants showed a markedly higher resistance against oxidative stress in comparison with the wildtype.

### Example 6 Analysis of the resistance of BoNT/A_Lc and BoNT/A_Lc mutants against cleavage by pronase

The reaction was performed in 25 mM Tris-HCl (pH 8) buffer at 37 °C. 25 µg of the corresponding BoNT/A-Lc mutant were incubated with 5 mU of pronase. A reaction volume of 200 µL was applied. Sampling of 35 µL was made after 0 h, 1 h, 2 h, 4 h, 8 h and 24 h. The different samples were analyzed by SDS-PAGE and compared via densitometric measurements. LC/A form WT was used as reference.

### Example 7 Analysis of the resistance of BoNT/A and BoNT/A mutants against cleavage by trypsin

The reaction was performed in 25 mM Tris-HCl (pH 8) buffer at 25 °C. 25 µg of the corresponding LC/A variants were incubated with 1 ng of trypsin. A reaction volume of 200 µL was applied. Sampling of 35 µL was made after 0 h, 1 h, 2 h, 4 h, 8 h and 24 h. The different samples were analyzed by SDS-PAGE and compared via densitometric measurements. LC/A form WT was used as reference.

### Example 8 Analysis of the resistance of BoNT/A and BoNT/A mutants against cleavage by µ-calpain

The reaction was performed in a reaction buffer containing 50 mM Hepes, 150 mM NaCl, 2 µM CaCl2 (pH 6,8) at 37 °C. 25 µg of the corresponding LC/A variants were incubated with 50 ng of µ Calpain (Sigma Alldrich). A reaction volume of 200 µL was applied. Sampling of 35 µL was made after 0 h, 1 h, 2 h, 4 h, 8 h and 24 h. The different samples were analyzed by SDS-PAGE and compared via densitometric measurements. LC/A form WT was used as reference.

### Example 9 Determination of the resistance light chain mutants against proteolytic degradation by trypsin, pronase and µ-calpain

The mutants were analyzed in the cleavage assay as described in Example 6 and 7 and 8. As an example figure 3, 4 and 5 show the behavior of the mutant BoNT/A_LC_Δ2627_Δ6465_Δ125 in the assay after analysis in SDS-PAGE. The degree of degradation was measured densitometrically. The results for all mutants are summarized in table 3 with respect to the following assessment:
+ > 50% degradation of Lc/A (detectable at ∼ 50 kDa)
+/- > 70% degradation of Lc/A (detectable at ∼ 50 kDa)
++ < 35% degradation of Lc/A (detectable at ∼ 50 kDa)

**Table 3.**

| Mutant | Resistance against pronase | Resistance against trypsin | Resistance Against µ-calpain |
|---|---|---|---|
| BoNT/A-Lc | + | + | n.d. |
| BoNT/A-Lc_Δ6465 | ++ | ++ | ++ |
| BoNT/A-Lc_Δ2627 | ++ | +/- | n.d. |
| BoNT/A-Lc_Δ122 | + | ++ | n.d. |
| BoNT/A-Lc_Δ2627_Δ6465 | ++ | ++ | n.d. |
| BoNT/A-Lc_Δ6465_Δ125 | + | ++ | n.d. |
| BoNT/A-Lc_Δ6465_Δ122 | + | ++ | n.d. |
| BoNT/A-Lc_Δ2627_Δ6465_Δ125 | ++ | ++ | ++ |
| BoNT/A-Lc_Δ6465_I138W/Y144W | ++ | ++ | n.d. |
| BoNT/A-Lc_Δ6465_E325W_F331W | ++ | ++ | n.d. |
| BoNT/A-Lc_Δ125 | ++ | ++ | n.d. |
| BoNT-Lc_I138W/Y144W | + | ++ | ++ |
| BoNT-Lc_Δ6465_I303C_L310C | ++ | ++ | ++ |
| BoNT-Lc_E325W_F331W | ++ | ++ | n.d. |
| BoNTLc_ I138W/Q139E/P140N/S143K/Y144W ΔD141 | ++ | ++ | ++ |
| BoNT-Lc_ E/P140N/S143K/Y ΔD141 | ++ | ++ | n.d. |
| BoNT-Lc_Δ6465_ I138W/Q139E/P140N/S143K/Y144W ΔD141 | ++ | ++ | ++ |
| BoNT-Lc_Δ6465_ /Q139E/P140N/S143K ΔD141 | ++ | ++ | n.d. |

### Example 10 Assessment of thermal stability by CD measurement

Thermal stability was assessed by thermal denaturation monitoring ellipticity (Circular Dichroism signal) at 222 nm between 20-60°C. Ellipticity curves were analyzed assuming a two-state model (protein undergoes unfolding transition between the folded and unfolded state). BoNT/A-Lc_Δ2627_Δ6465 and BoNT/A-Lc_Δ6465_Δ125 were analysed in comparison to the WT-Lc. (figure 7) Loop modification led to an increase of the mid point of thermal transition, Tm, respect to WT Lc. demonstrating a higher thermal stability of the mutants: Tm for the WT BoNT/A-Lc was at 38,9°C,whereas Tm for BoNT/A-Lc_Δ2627_Δ6465 was 39,6°C and Tm for BoNT/A-Lc_Δ6465_Δ125 was 42,1°C.

**Table 1: Sequences**

| (All sequences include a His-tag) |
|---|
| SEQ ID NO 1: recombinant BoNT/ A-Lc |
| SEQ ID NO 2: BoNT/A-Lc_M106I/M253L/M411F |
| SEQ ID NO 3 BoNT/A-Lc_M1061/M253V/M411F |
| SEQ ID NO 4: BoNT/A-Lc_M106I/M253V/M411K |
| SEQ ID NO 5: BoNT/A-Lc_M106L/M253L/M411Q |
| SEQ ID NO 6: BoNT/A-Lc_M106L/M253Y/M411K |
| SEQ ID NO 7: BoNT/A-Lc_M30F/M106L/C134I/C165I/M253L/M313I/M344L/M411Q |
| SEQ ID NO 8: BoNT/A-Lc_M30Y/M106L/C134I/M253L/M344L/M411Q |
| SEQ ID NO 9: BoNT/A-Lc_Δ2627 |
| SEQ ID NO 10: BoNT/A-Lc_Δ6465 |
| SEQ ID NO 11: BoNT/A-Lc_Δ125 |
| SEQ ID NO 12: BoNT/A-Lc_Δ122 |
| SEQ ID NO 13: BoNT/A-Lc-Δ6465_Δ125 |
| SEQ ID NO 14: BoNT/A-Lc_Δ2627_Δ6465_Δ125 |
| SEQ ID NO 15: BoNT/A-Lc_Δ6465_Δ122 |
| SEQ ID NO 16: BoNT/A-Lc_Δ2627_Δ122 |
| SEQ ID NO 17: BoNT/A-Lc_Δ6465_I138W/Y144W |
| SEQ ID NO 18: BoNT/A-Lc_E325W/Y331W |
| SEQ ID NO 19: BoNT/A-Lc_Δ6465 I303C_L310C |
| SEQ ID NO 20: BoNT/A-Lc_I138W_Q139E_ΔP140_D141N/S143KY144W |
| SEQ ID NO 21: BoNT/A-Lc_Δ6465_I138W_Q139E_ΔP140_D141N/S143KY144W |
| SEQ ID NO 22: BoNT/A-Lc_Δ6465_Q139E_ΔP140_D141N/S143K |

SEQ ID NO 1: recombinant BoNT/ A including His.tag 
MPFVNKQFNYKDPVNGVDIAYIKIPNAGQMQPVKAFKIHNKIWVIPERDTFTNPEEGDLNPP
PEAKQVPVSYYDSTYLSTDNEKDNYLKGVTKLFERIYSTDLGRMLLTSIVRGIPFWGGST
IDTELKVIDTNCINVIQPDGSYRSEELNLVIIGPSADIIQFECKSFGHEVLNLTRNGYGS
TQYIRFSPDFTFGFEESLEVDTNPLLGAGKFATDPAVTLAHELIHAGHRLYGIAINPNRV
FKVNTNAYYEMSGLEVSFEELRTFGGHDAKFIDSLQENEFRLYYYNKFKDIASTLNKAKS
IVGTTASLQYMKNVFKEKYLLSEDTSGKFSVDKLKFDKLYKMLTEIYTEDNFVKFFKVLN
RKTYLNFDKAVFKINIVPKVNYTIYDGFNLRNTNLAANFNGQNTEINNMNFTKLKNFTGL
FEFYKLLGSADLVPRGSANSSSVDKLWSHPQFEKLEHHHHHH
SEQ ID NO 2: BoNT/A-Lc_M106I/M253L/M411F 
MPFVNKQFNYKDPVNGVDIAYIKIPNAGQMQPVKAFKIHNKIWVIPERDTFTNPEEGDLN
PPPEAKQVPVSYYDSTYLSTDNEKDNYLKGVTKLFERIYSTDLGRILLTSIVRGIPFWGG
STIDTELKVIDTNCINVIQPDGSYRSEELNLVIIGPSADIIQFECKSFGHEVLNLTRNGY
GSTQYIRFSPDFTFGFEESLEVDTNPLLGAGKFATDPAVTLAHELIHAGHRLYGIAINPN
RVFKVNTNAYYELSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKV
 
LNRKTYLNFDKAVFKINIVPKVNYTIYDGFNLRNTNLAANFNGQNTEINNFNFTKLKNFT
GLFEFYKLLGSADLVPRGSANSSSVDKLWSHPQFEKLEHHHHHH
SEQ NO 3 BoNT/A-Lc_M106I/M253V/M411F 
MPFVNKQFNYKDPVNGVDIAYIKIPNAGQMQPVKAFKIHNKIWVIPERDTFTNPEEGDLN

PPPEAKQVPVSYYDSTYLSTDNEKDNYLKGVTKLFERIYSTDLGRILLTSIVRGIPFWGG

STIDTELKVIDTNCINVIQPDGSYRSEELNLVIIGPSADIIQFECKSFGHEVLNLTRNGY

GSTQYIRFSPDFTFGFEESLEVDTNPLLGAGKFATDPAVTLAHELIHAGHRLYGIAINPN

RVFKVNTNAYYEVSGLEVSFEELRTFGGHDAKFIDSLQENEFRLYYYNKFKDIASTLNKA

KSIVGTTASLQYMKNVFKEKYLLSEDTSGKFSVDKLKFDKLYKMLTEIYTEDNFVKFFKV

LNRKTYLNFDKAVFKINIVPKVNYTIYDGFNLRNTNLAANFNGQNTEINNFNFTKLKNFT

GLFEFYKLLGSADLVPRGSANSSSVDKLWSHPQFEKLEHHHHHH
SEQ ID NO 4: BoNT/A-Lc_M106I, M253V, M411 K 
MPFVNKQFNYKDPVNGVDIAYIKIPNAGQMQPVKAFKIHNKIWVIPERDTFTNPEEGDLN

PPPEAKQVPVSYYDSTYLSTDNEKDNYLKGVTKLFERIYSTDLGRILLTSIVRGIPFWGG

STIDTELKVIDTNCINVIQPDGSYRSEELNLVIIGPSADIIQFECKSFGHEVLNLTRNGY

GSTQYIRFSPDFTFGFEESLEVDTNPLLGAGKFATDPAVTLAHELIHAGHRLYGIAINPN

RVFKVNTNAYYEVSGLEVSFEELRTFGGHDAKFIDSLQENEFRLYYYNKFKDIASTLNKA

KSIVGTTASLQYMKNVFKEKYLLSEDTSGKFSVDKLKFDKLYKMLTEIYTEDNFVKFFKV

LNRKTYLNFDKAVFKINIVPKVNYTIYDGFNLRNTNLAANFNGQNTEINNKNFTKLKNFT

GLFEFYKLLGSADLVPRGSANSSSVDKLWSHPQFEKLEHHHHHH
SEQ ID NO 5: BoNT/A-Lc_M106L/M253L/M411Q 
MPFVNKQFNYKDPVNGVDIAYIKIPNAGQMQPVKAFKIHNKIWVIPERDTFTNPEEGDLN
PPPEAKQVPVSYYDSTYLSTDNEKDNYLKGVTKLFERIYSTDLGRLLLTSIVRGIPFWGG
STIDTELKVIDTNCINVIQPDGSYRSEELNLVIIGPSADIIQFECKSFGHEVLNLTRNGY
GSTQYIRFSPDFTFGFEESLEVDTNPLLGAGKFATDPAVTLAHELIHAGHRLYGIAINPN
RVFKVNTNAYYELSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKV
LNRKTYLNFDKAVFKINIVPKVNYTIYDGFNLRNTNLAANFNGQNTEINNQNFTKLKNFT
GLFEFYKLLGSADLVPRGSANSSSVDKLWSHPQFEKLEHHHHHH
SEQ ID NO 6: BoNT/A-Lc_M106L/M253Y/M411 K 
MPFVNKQFNYKDPVNGVDIAYIKIPNAGQMQPVKAFKIHNKIWVIPERDTFTNPEEGDLN
PPPEAKQVPVSYYDSTYLSTDNEKDNYLKGVTKLFERIYSTDLGRLLLTSIVRGIPFWGG
STIDTELKVIDTNCINVIQPDGSYRSEELNLVIIGPSADIIQFECKSFGHEVLNLTRNGY
GSTQYIRFSPDFTFGFEESLEVDTNPLLGAGKFATDPAVTLAHELIHAGHRLYGIAINPN
RVFKVNTNAYYEYSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKV
LNRKTYLNFDKAVFKINIVPKVNYTIYDGFNLRNTNLAANFNGQNTEINNKNFTKLKNFT
GLFEFYKLLGSADLVPRGSANSSSVDKLWSHPQFEKLEHHHHHH
SEQ ID NO 7: BoNT/A-Lc_M30F/M106L/C134I/C165I/M253L/M313I/M344L/M411Q 
MPFVNKQFNYKDPVNGVDIAYIKIPNAGQFQPVKAFKIHNKIWVIPERDTFTNPEEGDLN

PPPEAKQVPVSYYDSTYLSTDNEKDNYLKGVTKLFERIYSTDLGRLLLTSIVRGIPFWGG

STIDTELKVIDTNIINVIQPDGSYRSEELNLVIIGPSADIIQFEIKSFGHEVLNLTRNGY

GSTQYIRFSPDFTFGFEESLEVDTNPLLGAGKFATDPAVTLAHELIHAGHRLYGIAINPN

RVFKVNTNAYYELSGLEVSFEELRTFGGHDAKFIDSLQENEFRLYYYNKFKDIASTLNKA

KSIVGTTASLQYIKNVFKEKYLLSEDTSGKFSVDKLKFDKLYKLLTEIYTEDNFVKFFKV

LNRKTYLNFDKAVFKINIVPKVNYTIYDGFNLRNTNLAANFNGQNTEINNQNFTKLKNFT

GLFEFYKLLGSADLVPRGSANSSSVDKLWSHPQFEKLEHHHHHH
SEQ ID NO 8: BoNT/A-Lc_M30Y/M106L/C134I/M253L/M344L/M411Q 
MPFVNKQFNYKDPVNGVDIAYIKIPNAGQYQPVKAFKIHNKIWVIPERDTFTNPEEGDLN
PPPEAKQVPVSYYDSTYLSTDNEKDNYLKGVTKLFERIYSTDLGRLLLTSIVRGIPFWGG
STIDTELKVIDTNIINVIQPDGSYRSEELNLVIIGPSADIIQFECKSFGHEVLNLTRNGY
GSTQYIRFSPDFTFGFEESLEVDTNPLLGAGKFATDPAVTLAHELIHAGHRLYGIAINPN
RVFKVNTNAYYELSGLEVSFEELRTFGGHDAKFIDSLQENEFRLYYYNKFKDIASTLNKA
KSIVGTTASLQYIKNVFKEKYLLSEDTSGKFSVDKLKFDKLYKLLTEIYTEDNFVKFFKV
LNRKTYLNFDKAVFKINIVPKVNYTIYDGFNLRNTNLAANFNGQNTEINNQNFTKLKNFT
GLFEFYKLLGSADLVPRGSANSSSVDKLWSHPQFEKLEHHHHHH
SEQ ID NO 9: BoNT/A-Lc_Δ2627 
MPFVNKQFNYKDPVNGVDIAYIKIPGQMQPVKAFKIHNKIWVIPERDTFTNPEEGDLNPP

PEAKQVPVSYYDSTYLSTDNEKDNYLKGVTKLFERIYSTDLGRMLLTSIVRGIPFWGGST
 
IDTELKVIDTNCINVIQPDGSYRSEELNLVIIGPSADIIQFECKSFGHEVLNLTRNGYGS

TQYIRFSPDFTFGFEESLEVDTNPLLGAGKFATDPAVTLAHELIHAGHRLYGIAINPNRV

FKVNTNAYYEMSGLEVSFEELRTFGGHDAKFIDSLQENEFRLYYYNKFKDIASTLNKAKS

IVGTTASLQYMKNVFKEKYLLSEDTSGKFSVDKLKFDKLYKMLTEIYTEDNFVKFFKVLN

RKTYLNFDKAVFKINIVPKVNYTIYDGFNLRNTNLAANFNGQNTEINNMNFTKLKNFTGL

FEFYKLLGSADLVPRGSANSSSVDKLWSHPQFEKLEHHHHHH
SEQ ID NO 10: BoNT/A-Lc_Δ6465 
MPFVNKQFNYKDPVNGVDIAYIKIPNAGQMQPVKAFKIHNKIWVIPERDTFTNPEEGDLN
PPPKQVPVSYYDSTYLSTDNEKDNYLKGVTKLFERIYSTDLGRMLLTSIVRGIPFWGGST
IDTELKVIDTNCINVIQPDGSYRSEELNLVIIGPSADIIQFECKSFGHEVLNLTRNGYGS
TQYIRFSPDFTFGFEESLEVDTNPLLGAGKFATDPAVTLAHELIHAGHRLYGIAINPNRV
FKVNTNAYYEMSGLEVSFEELRTFGGHDAKFIDSLQENEFRLYYYNKFKDIASTLNKAKS
IVGTTASLQYMKNVFKEKYLLSEDTSGKFSVDKLKFDKLYKMLTEIYTEDNFVKFFKVLN
RKTYLNFDKAVFKINIVPKVNYTIYDGFNLRNTNLAANFNGQNTEINNMNFTKLKNFTGL
FEFYKLLGSADLVPRGSANSSSVDKLWSHPQFEKLEHHHHHH
SEQ ID NO 11: BoNT/A-Lc_Δ125 
MPFVNKQFNYKDPVNGVDIAYIKIPNAGQMQPVKAFKIHNKIWVIPERDTFTNPEEGDLN

PPPEAKQVPVSYYDSTYLSTDNEKDNYLKGVTKLFERIYSTDLGRMLLTSIVRGIPFWGG

STIDELKVIDTNCINVIQPDGSYRSEELNLVIIGPSADIIQFECKSFGHEVLNLTRNGYG

STQYIRFSPDFTFGFEESLEVDTNPLLGAGKFATDPAVTLAHELIHAGHRLYGIAINPNR

VFKVNTNAYYEMSGLEVSFEELRTFGGHDAKFIDSLQENEFRLYYYNKFKDIASTLNKAK
 
SIVGTTASLQYMKNVFKEKYLLSEDTSGKFSVDKLKFDKLYKMLTEIYTEDNFVKFFKVL
NRKTYLNFDKAVFKINIVPKVNYTIYDGFNLRNTNLAANFNGQNTEINNMNFTKLKNFTG
LFEFYKLLGSADLVPRGSANSSSVDKLWSHPQFEKLEHHHHHH
SEQ ID NO 12: BoNT/A-Lc_Δ122 
MPFVNKQFNYKDPVNGVDIAYIKIPNAGQMQPVKAFKIHNKIWVIPERDTFTNPEEGDLN

PPPEAKQVPVSYYDSTYLSTDNEKDNYLKGVTKLFERIYSTDLGRMLLTSIVRGIPFWGG

SIDTELKVIDTNCINVIQPDGSYRSEELNLVIIGPSADIIQFECKSFGHEVLNLTRNGYG

STQYIRFSPDFTFGFEESLEVDTNPLLGAGKFATDPAVTLAHELIHAGHRLYGIAINPNR

VFKVNTNAYYEMSGLEVSFEELRTFGGHDAKFIDSLQENEFRLYYYNKFKDIASTLNKAK

SIVGTTASLQYMKNVFKEKYLLSEDTSGKFSVDKLKFDKLYKMLTEIYTEDNFVKFFKVL

NRKTYLNFDKAVFKINIVPKVNYTIYDGFNLRNTNLAANFNGQNTEINNMNFTKLKNFTG

LFEFYKLLGSADLVPRGSANSSSVDKLWSHPQFEKLEHHHHHH
SEQ ID NO 13: BoNT/A-Lc_Δ6465_Δ125 
MPFVNKQFNYKDPVNGVDIAYIKIPNAGQMQPVKAFKIHNKIWVIPERDTFTNPEEGDLN

PPPKQVPVSYYDSTYLSTDNEKDNYLKGVTKLFERIYSTDLGRMLLTSIVRGIPFWGGST

IDELKVIDTNCINVIQPDGSYRSEELNLVIIGPSADIIQFECKSFGHEVLNLTRNGYGST

QYIRFSPDFTFGFEESLEVDTNPLLGAGKFATDPAVTLAHELIHAGHRLYGIAINPNRVF

KVNTNAYYEMSGLEVSFEELRTFGGHDAKFIDSLQENEFRLYYYNKFKDIASTLNKAKSI
 
VGTTASLQYMKNVFKEKYLLSEDTSGKFSVDKLKFDKLYKMLTEIYTEDNFVKFFKVLNR
KTYLNFDKAVFKINIVPKVNYTIYDGFNLRNTNLAANFNGQNTEINNMNFTKLKNFTGLF
EFYKLLGSADLVPRGSANSSSVDKLWSHPQFEKLEHHHHHH
SEQ ID NO 14: BoNT/A-Lc_Δ2627_Δ6465_Δ125 
MPFVNKQFNYKDPVNGVDIAYIKIPGQMQPVKAFKIHNKIWVIPERDTFTNPEEGDLN

PPPKQVPVSYYDSTYLSTDNEKDNYLKGVTKLFERIYSTDLGRMLLTSIVRGIPFWGGST

IDELKVIDTNCINVIQPDGSYRSEELNLVIIGPSADIIQFECKSFGHEVLNLTRNGYGST

QYIRFSPDFTFGFEESLEVDTNPLLGAGKFATDPAVTLAHELIHAGHRLYGIAINPNRVF

KVNTNAYYEMSGLEVSFEELRTFGGHDAKFIDSLQENEFRLYYYNKFKDIASTLNKAKSI

VGTTASLQYMKNVFKEKYLLSEDTSGKFSVDKLKFDKLYKMLTEIYTEDNFVKFFKVLNR

KTYLNFDKAVFKINIVPKVNYTIYDGFNLRNTNLAANFNGQNTEINNMNFTKLKNFTGLF

EFYKLLGSADLVPRGSANSSSVDKLWSHPQFEKLEHHHHHH
SEQ ID NO 15: BoNT/A-Lc_Δ6465_Δ122 
MPFVNKQFNYKDPVNGVDIAYIKIPNAGQMQPVKAFKIHNKIWVIPERDTFTNPEEGDLN
PPPKQVPVSYYDSTYLSTDNEKDNYLKGVTKLFERIYSTDLGRMLLTSIVRGIPFWGGSI
DTELKVIDTNCINVIQPDGSYRSEELNLVIIGPSADIIQFECKSFGHEVLNLTRNGYGST
QYIRFSPDFTFGFEESLEVDTNPLLGAGKFATDPAVTLAHELIHAGHRLYGIAINPNRVF
KVNTNAYYEMSGLEVSFEELRTFGGHDAKFIDSLQENEFRLYYYNKFKDIASTLNKAKSI
VGTTASLQYMKNVFKEKYLLSEDTSGKFSVDKLKFDKLYKMLTEIYTEDNFVKFFKVLNR
KTYLNFDKAVFKINIVPKVNYTIYDGFNLRNTNLAANFNGQNTEINNMNFTKLKNFTGLF
 EFYKLLGSADLVPRGSANSSSVDKLWSHPQFEKLEHHHHHH
SEQ ID NO 16: BoNT/A-Lc_Δ2627_Δ122 
MPFVNKQFNYKDPVNGVDIAYIKIPGQMQPVKAFKIHNKIWVIPERDTFTNPEEGDLNPP
PEAKQVPVSYYDSTYLSTDNEKDNYLKGVTKLFERIYSTDLGRMLLTSIVRGIPFWGGS
IDTELKVIDTNCINVIQPDGSYRSEELNLVIIGPSADIIQFECKSFGHEVLNLTRNGYGS
TQYIRFSPDFTFGFEESLEVDTNPLLGAGKFATDPAVTLAHELIHAGHRLYGIAINPNRV
FKVNTNAYYEMSGLEVSFEELRTFGGHDAKFIDSLQENEFRLYYYNKFKDIASTLNKAKS
IVGTTASLQYMKNVFKEKYLLSEDTSGKFSVDKLKFDKLYKMLTEIYTEDNFVKFFKVLN
RKTYLNFDKAVFKINIVPKVNYTIYDGFNLRNTNLAANFNGQNTEINNMNFTKLKNFTGL
FEFYKLLGSADLVPRGSANSSSVDKLWSHPQFEKLEHHHHHH
SEQ ID NO 17: BoNT/A-Lc_Δ6465_I138W/Y144W 
MPFVNKQFNYKDPVNGVDIAYIKIPNAGQMQPVKAFKIHNKIWVIPERDTFTNPEEGDLN
PPPKQVPVSYYDSTYLSTDNEKDNYLKGVTKLFERIYSTDLGRMLLTSIVRGIPFWGGST
IDTELKVIDTNCINVWQPDGSWRSEELNLVIIGPSADIIQFECKSFGHEVLNLTRNGYGS
TQYIRFSPDFTFGFEESLEVDTNPLLGAGKFATDPAVTLAHELIHAGHRLYGIAINPNRV
FKVNTNAYYEMSGLEVSFEELRTFGGHDAKFIDSLQENEFRLYYYNKFKDIASTLNKAKS
IVGTTASLQYMKNVFKEKYLLSEDTSGKFSVDKLKFDKLYKMLTEIYTEDNFVKFFKVLN
RKTYLNFDKAVFKINIVPKVNYTIYDGFNLRNTNLAANFNGQNTEINNMNFTKLKNFTGL
FEFYKLLGSADLVPRGSANSSSVDKLWSHPQFEKLEHHHHHH
SEQ ID NO 18: BoNT/A-Lc_E325W/Y331W 
MPFVNKQFNYKDPVNGVDIAYIKIPNAGQMQPVKAFKIHNKIWVIPERDTFTNPEEGDLN

PPPEAKQVPVSYYDSTYLSTDNEKDNYLKGVTKLFERIYSTDLGRMLLTSIVRGIPFWGG

STIDTELKVIDTNCINVIQPDGSYRSEELNLVIIGPSADIIQFECKSFGHEVLNLTRNGY

GSTQYIRFSPDFTFGFEESLEVDTNPLLGAGKFATDPAVTLAHELIHAGHRLYGIAINPN

RVFKVNTNAYYEMSGLEVSFEELRTFGGHDAKFIDSLQENEFRLYYYNKFKDIASTLNKA

KSIVGTTASLQYMKNVFKEKYLLSWDTSGKWSVDKLKFDKLYKMLTEIYTEDNFVKFFKV

LNRKTYLNFDKAVFKINIVPKVNYTIYDGFNLRNTNLAANFNGQNTEINNMNFTKLKNFT

GLFEFYKLLGSADLVPRGSANSSSVDKLWSHPQFEKLEHHHHHH
SEQ ID NO 19: BoNT/A-Lc_Δ6465_I303C_L310C 
MPFVNKQFNYKDPVNGVDIAYIKIPNAGQMQPVKAFKIHNKIWVIPERDTFTNPEEGDLN
PPPKQVPVSYYDSTYLSTDNEKDNYLKGVTKLFERIYSTDLGRMLLTSIVRGIPFWGGST
IDTELKVIDTNCINVIQPDGSYRSEELNLVIIGPSADIIQFECKSFGHEVLNLTRNGYGS
TQYIRFSPDFTFGFEESLEVDTNPLLGAGKFATDPAVTLAHELIHAGHRLYGIAINPNRV
FKVNTNAYYEMSGLEVSFEELRTFGGHDAKFIDSLQENEFRLYYYNKFKDIASTLNKAKS
CVGTTASCQYMKNVFKEKYLLSEDTSGKFSVDKLKFDKLYKMLTEIYTEDNFVKFFKVLN
RKTYLNFDKAVFKINIVPKVNYTIYDGFNLRNTNLAANFNGQNTEINNMNFTKLKNFTGL
FEFYKLLGSADLVPRGSANSSSVDKLWSHPQFEKLEHHHHHH
SEQ ID NO 20: BoNT/A-Lc_I138W_Q139E_Δ140_D141N/S143KY144W 
MPFVNKQFNYKDPVNGVDIAYIKIPNAGQMQPVKAFKIHNKIWVIPERDTFTNPEEGDLN

PPPEAKQVPVSYYDSTYLSTDNEKDNYLKGVTKLFERIYSTDLGRMLLTSIVRGIPFWGG

STIDTELKVIDTNCINVWENGKWRSEELNLVIIGPSADIIQFECKSFGHEVLNLTRNGYG

STQYIRFSPDFTFGFEESLEVDTNPLLGAGKFATDPAVTLAHELIHAGHRLYGIAINPNR

VFKVNTNAYYEMSGLEVSFEELRTFGGHDAKFIDSLQENEFRLYYYNKFKDIASTLNKAK

SIVGTTASLQYMKNVFKEKYLLSEDTSGKFSVDKLKFDKLYKMLTEIYTEDNFVKFFKVL

NRKTYLNFDKAVFKINIVPKVNYTIYDGFNLRNTNLAANFNGQNTEINNMNFTKLKNFTG

LFEFYKLLGSADLVPRGSANSSSVDKLWSHPQFEKLEHHHHHH
SEQ ID NO 21: BoNT/A-Lc_Δ6465_I138W_Q139E_Δ140_D141N/S143KY144W 
MPFVNKQFNYKDPVNGVDIAYIKIPNAGQMQPVKAFKIHNKIWVIPERDTFTNPEEGDLN
PPPKQVPVSYYDSTYLSTDNEKDNYLKGVTKLFERIYSTDLGRMLLTSIVRGIPFWGGST
IDTELKVIDTNCINVWENGKWRSEELNLVIIGPSADIIQFECKSFGHEVLNLTRNGYGST
QYIRFSPDFTFGFEESLEVDTNPLLGAGKFATDPAVTLAHELIHAGHRLYGIAINPNRVF
KVNTNAYYEMSGLEVSFEELRTFGGHDAKFIDSLQENEFRLYYYNKFKDIASTLNKAKSI
VGTTASLQYMKNVFKEKYLLSEDTSGKFSVDKLKFDKLYKMLTEIYTEDNFVKFFKVLNR
KTYLNFDKAVFKINIVPKVNYTIYDGFNLRNTNLAANFNGQNTEINNMNFTKLKNFTGLF
EFYKLLGSADLVPRGSANSSSVDKLWSHPQFEKLEHHHHHH
SEQ ID NO 22: BoNT/A-Lc_Δ6465_Q139E_ΔP140_D141N/S143K

MPFVNKQFNYKDPVNGVDIAYIKIPNAGQMQPVKAFKIHNKIWVIPERDTFTNPEEGDLN

PPPKQVPVSYYDSTYLSTDNEKDNYLKGVTKLFERIYSTDLGRMLLTSIVRGIPFWGGST
IDTELKVIDTNCINVIENGKYRSEELNLVIIGPSADIIQFECKSFGHEVLNLTRNGYGST
QYIRFSPDFTFGFEESLEVDTNPLLGAGKFATDPAVTLAHELIHAGHRLYGIAINPNRVF
KVNTNAYYEMSGLEVSFEELRTFGGHDAKFIDSLQENEFRLYYYNKFKDIASTLNKAKSI
VGTTASLQYMKNVFKEKYLLSEDTSGKFSVDKLKFDKLYKMLTEIYTEDNFVKFFKVLNR
KTYLNFDKAVFKINIVPKVNYTIYDGFNLRNTNLAANFNGQNTEINNMNFTKLKNFTGLF
EFYKLLGSADLVPRGSANSSSVDKLWSHPQFEKLEHHHHHH

## Claims

1. A recombinant botulinum neurotoxin serotype A or a functional variant thereof comprising at least one amino acid modification, wherein said at least one amino acid modification is located in the light chain at position 1 to 438 (SEQ ID NO 1), wherein said at least one amino acid modification confers a higher oxidative resistance and/or higher stability against proteolytic degradation of the light chain than an otherwise identical botulinum toxin light chain lacking said at least one amino acid modification.

2. The recombinant neurotoxin of claim 1, wherein said at least one amino acid modification is located in the light chain at at least one position selected from N26, A27, M30, E64, A65, M106, T125, C134, 1138, Q139, P140, D141, S143, Y144, C165, M253L, S302, I303, S309, L310, E325, M313, F331, M344 and M411.

3. The recombinant neurotoxin of claim 1 or 2, wherein the recombinant neurotoxin comprises at least two or three or four or five or six or seven or eight amino acid modifications.

4. The recombinant neurotoxin of any one of claim 1 to 3, wherein three amino acid modifications are located in the light chain at position M106, M253, M411, in particular wherein these amino acids are substituted as follows M106L, M253L, M411Q.

5. The recombinant neurotoxin of any one of claim 1 to 3, wherein six amino acid modifications are located in the light chain at position M30, M106, C134, M253, M344 and M411, in particular wherein these amino acids are substituted as follows M30Y, M106L, C134I, M253L, M344L, M411 Q.

6. The recombinant neurotoxin of any one of claim 1 to 3, wherein eight amino acid modifications are located in the light chain at position M30, M106, C134, C165, M253, M313, M344, M411, in particular wherein these amino acids are substituted as follows M30F, M106L, C134I, C165I, M253L, M313I, M344L and M411 Q.

7. The recombinant neurotoxin of any one of claim 1 to 3, wherein eight amino acid modifications are located in the light chain at position M30, M106, C134, C165, M253, M313, M344, M411 wherein these amino acids are substituted as follows M30F, M106L, C134I, C165I, M253V, M3131, M344L, and M411 F.

8. The recombinant neurotoxin of any one of claim 1 to 3, wherein eight amino acid modifications are located in the light chain at position M30, M106, C134, C165, M253, M313, M344, M411 wherein these amino acids are substituted as follows M30F, M106L, C134I, C165I, M253Y, M3131, M344L and M411 K.

9. The recombinant neurotoxin of any one of claim 1 to 3, wherein one or more of the amino acids at position N26, A27, E64, A65,T125 are deleted.

10. The recombinant neurotoxin of any one of claim 1 to 3, wherein amino acid modifications are located in the light chain at position 1138, Y144, I303, L310, E325, F331, S302C and S309C, wherein these amino acids are pairwise substituted as follows I138W and Y144W, E325W and F331W, S302C and S309C, I303C and L310C.

11. The recombinant neurotoxin of any one of claim 1 to 3, wherein amino acid modifications are located in the light chain at position 1138 Q139, P140, D141 S143 and Y144, in particular wherein D141 is deleted and the other amino acids are substituted as follows I38W, Q139E, P140N, S143K and Y144W.

12. A composition comprising the recombinant neurotoxin of any one of claims 1 to 11.

13. A pharmaceutical composition comprising the recombinant neurotoxin of any one of claims 1 to 11.

14. Use of the recombinant neurotoxin of any one of claims 1 to 11 for cosmetic treatment.

15. A method for the generation of a recombinant neurotoxin according to any one of claims 1 to 11, comprising the step of obtaining a recombinant nucleic acid sequence encoding a recombinant single-chain precursor neurotoxin by modification of the nucleic acid sequence encoding a parental neurotoxin, or wherein said method further comprises the step of heterologously expressing said recombinant nucleic acid sequence in a host cell, particularly in a bacterial host cell, more particularly in an *E. coli* host cell.


SEQUENCE LISTING

<110> Merz Pharma GmbH & Co. KGaA

<120> NOVEL RECOMBINANT BOTULINUM NEUROTOXINS WITH A STABILIZED LIGHT CHAIN

<130> 1316-000-EP

<160> 22

<170> BiSSAP 1.3.5

<210> 1
<211> 464
<212> PRT
<213> Artificial Sequence

<223> "recombinant BoNT/ A including His-tag"

<220>
<223> recombinant BoNT/ A including His-tag

<400> 1
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Met Leu Leu Thr Ser Ile Val
            100                 105                 110
Arg Gly Ile Pro Phe Trp Gly Gly Ser Thr Ile Asp Thr Glu Leu Lys
        115                 120                 125
Val Ile Asp Thr Asn Cys Ile Asn Val Ile Gln Pro Asp Gly Ser Tyr
    130                 135                 140
Arg Ser Glu Glu Leu Asn Leu Val Ile Ile Gly Pro Ser Ala Asp Ile
145                 150                 155                 160
Ile Gln Phe Glu Cys Lys Ser Phe Gly His Glu Val Leu Asn Leu Thr
                165                 170                 175
Arg Asn Gly Tyr Gly Ser Thr Gln Tyr Ile Arg Phe Ser Pro Asp Phe
            180                 185                 190
Thr Phe Gly Phe Glu Glu Ser Leu Glu Val Asp Thr Asn Pro Leu Leu
        195                 200                 205
Gly Ala Gly Lys Phe Ala Thr Asp Pro Ala Val Thr Leu Ala His Glu
    210                 215                 220
Leu Ile His Ala Gly His Arg Leu Tyr Gly Ile Ala Ile Asn Pro Asn
225                 230                 235                 240
Arg Val Phe Lys Val Asn Thr Asn Ala Tyr Tyr Glu Met Ser Gly Leu
                245                 250                 255
Glu Val Ser Phe Glu Glu Leu Arg Thr Phe Gly Gly His Asp Ala Lys
            260                 265                 270
Phe Ile Asp Ser Leu Gln Glu Asn Glu Phe Arg Leu Tyr Tyr Tyr Asn
        275                 280                 285
Lys Phe Lys Asp Ile Ala Ser Thr Leu Asn Lys Ala Lys Ser Ile Val
    290                 295                 300
Gly Thr Thr Ala Ser Leu Gln Tyr Met Lys Asn Val Phe Lys Glu Lys
305                 310                 315                 320

Tyr Leu Leu Ser Glu Asp Thr Ser Gly Lys Phe Ser Val Asp Lys Leu
                325                 330                 335
Lys Phe Asp Lys Leu Tyr Lys Met Leu Thr Glu Ile Tyr Thr Glu Asp
            340                 345                 350
Asn Phe Val Lys Phe Phe Lys Val Leu Asn Arg Lys Thr Tyr Leu Asn
        355                 360                 365
Phe Asp Lys Ala Val Phe Lys Ile Asn Ile Val Pro Lys Val Asn Tyr
    370                 375                 380
Thr Ile Tyr Asp Gly Phe Asn Leu Arg Asn Thr Asn Leu Ala Ala Asn
385                 390                 395                 400
Phe Asn Gly Gln Asn Thr Glu Ile Asn Asn Met Asn Phe Thr Lys Leu
                405                 410                 415
Lys Asn Phe Thr Gly Leu Phe Glu Phe Tyr Lys Leu Leu Gly Ser Ala
            420                 425                 430
Asp Leu Val Pro Arg Gly Ser Ala Asn Ser Ser Ser Val Asp Lys Leu
        435                 440                 445
Trp Ser His Pro Gln Phe Glu Lys Leu Glu His His His His His His
    450                 455                 460

<210> 2
<211> 464
<212> PRT
<213> Artificial Sequence

<223> "BoNT/A-Lc_ M106I/M253L/M411F including His-tag"

<220>
<223> BoNT/A-Lc_ M106I/M253L/M411F including His-tag

<400> 2
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Ile Leu Leu Thr Ser Ile Val
            100                 105                 110
Arg Gly Ile Pro Phe Trp Gly Gly Ser Thr Ile Asp Thr Glu Leu Lys
        115                 120                 125
Val Ile Asp Thr Asn Cys Ile Asn Val Ile Gln Pro Asp Gly Ser Tyr
    130                 135                 140
Arg Ser Glu Glu Leu Asn Leu Val Ile Ile Gly Pro Ser Ala Asp Ile
145                 150                 155                 160
Ile Gln Phe Glu Cys Lys Ser Phe Gly His Glu Val Leu Asn Leu Thr
                165                 170                 175
Arg Asn Gly Tyr Gly Ser Thr Gln Tyr Ile Arg Phe Ser Pro Asp Phe
            180                 185                 190
Thr Phe Gly Phe Glu Glu Ser Leu Glu Val Asp Thr Asn Pro Leu Leu
        195                 200                 205
Gly Ala Gly Lys Phe Ala Thr Asp Pro Ala Val Thr Leu Ala His Glu
    210                 215                 220
Leu Ile His Ala Gly His Arg Leu Tyr Gly Ile Ala Ile Asn Pro Asn
225                 230                 235                 240
Arg Val Phe Lys Val Asn Thr Asn Ala Tyr Tyr Glu Leu Ser Gly Leu

245                 250                 255
Glu Val Ser Phe Glu Glu Leu Arg Thr Phe Gly Gly His Asp Ala Lys
            260                 265                 270
Phe Ile Asp Ser Leu Gln Glu Asn Glu Phe Arg Leu Tyr Tyr Tyr Asn
        275                 280                 285
Lys Phe Lys Asp Ile Ala Ser Thr Leu Asn Lys Ala Lys Ser Ile Val
    290                 295                 300
Gly Thr Thr Ala Ser Leu Gln Tyr Met Lys Asn Val Phe Lys Glu Lys
305                 310                 315                 320
Tyr Leu Leu Ser Glu Asp Thr Ser Gly Lys Phe Ser Val Asp Lys Leu
                325                 330                 335
Lys Phe Asp Lys Leu Tyr Lys Met Leu Thr Glu Ile Tyr Thr Glu Asp
            340                 345                 350
Asn Phe Val Lys Phe Phe Lys Val Leu Asn Arg Lys Thr Tyr Leu Asn
        355                 360                 365
Phe Asp Lys Ala Val Phe Lys Ile Asn Ile Val Pro Lys Val Asn Tyr
    370                 375                 380
Thr Ile Tyr Asp Gly Phe Asn Leu Arg Asn Thr Asn Leu Ala Ala Asn
385                 390                 395                 400
Phe Asn Gly Gln Asn Thr Glu Ile Asn Asn Phe Asn Phe Thr Lys Leu
                405                 410                 415
Lys Asn Phe Thr Gly Leu Phe Glu Phe Tyr Lys Leu Leu Gly Ser Ala
            420                 425                 430
Asp Leu Val Pro Arg Gly Ser Ala Asn Ser Ser Ser Val Asp Lys Leu
        435                 440                 445
Trp Ser His Pro Gln Phe Glu Lys Leu Glu His His His His His His
    450                 455                 460

<210> 3
<211> 464
<212> PRT
<213> Artificial Sequence

<223> "BoNT/A-Lc_ M106I/M253V/ M411F including His-tag"

<220>
<223> BoNT/A-Lc_ M106I/M253V/ M411F including His-tag

<400> 3
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Ile Leu Leu Thr Ser Ile Val
            100                 105                 110
Arg Gly Ile Pro Phe Trp Gly Gly Ser Thr Ile Asp Thr Glu Leu Lys
        115                 120                 125
Val Ile Asp Thr Asn Cys Ile Asn Val Ile Gln Pro Asp Gly Ser Tyr
    130                 135                 140
Arg Ser Glu Glu Leu Asn Leu Val Ile Ile Gly Pro Ser Ala Asp Ile
145                 150                 155                 160
Ile Gln Phe Glu Cys Lys Ser Phe Gly His Glu Val Leu Asn Leu Thr
                165                 170                 175

Arg Asn Gly Tyr Gly Ser Thr Gln Tyr Ile Arg Phe Ser Pro Asp Phe
            180                 185                 190
Thr Phe Gly Phe Glu Glu Ser Leu Glu Val Asp Thr Asn Pro Leu Leu
        195                 200                 205
Gly Ala Gly Lys Phe Ala Thr Asp Pro Ala Val Thr Leu Ala His Glu
    210                 215                 220
Leu Ile His Ala Gly His Arg Leu Tyr Gly Ile Ala Ile Asn Pro Asn
225                 230                 235                 240
Arg Val Phe Lys Val Asn Thr Asn Ala Tyr Tyr Glu Val Ser Gly Leu
                245                 250                 255
Glu Val Ser Phe Glu Glu Leu Arg Thr Phe Gly Gly His Asp Ala Lys
            260                 265                 270
Phe Ile Asp Ser Leu Gln Glu Asn Glu Phe Arg Leu Tyr Tyr Tyr Asn
        275                 280                 285
Lys Phe Lys Asp Ile Ala Ser Thr Leu Asn Lys Ala Lys Ser Ile Val
    290                 295                 300
Gly Thr Thr Ala Ser Leu Gln Tyr Met Lys Asn Val Phe Lys Glu Lys
305                 310                 315                 320
Tyr Leu Leu Ser Glu Asp Thr Ser Gly Lys Phe Ser Val Asp Lys Leu
                325                 330                 335
Lys Phe Asp Lys Leu Tyr Lys Met Leu Thr Glu Ile Tyr Thr Glu Asp
            340                 345                 350
Asn Phe Val Lys Phe Phe Lys Val Leu Asn Arg Lys Thr Tyr Leu Asn
        355                 360                 365
Phe Asp Lys Ala Val Phe Lys Ile Asn Ile Val Pro Lys Val Asn Tyr
    370                 375                 380
Thr Ile Tyr Asp Gly Phe Asn Leu Arg Asn Thr Asn Leu Ala Ala Asn
385                 390                 395                 400
Phe Asn Gly Gln Asn Thr Glu Ile Asn Asn Phe Asn Phe Thr Lys Leu
                405                 410                 415
Lys Asn Phe Thr Gly Leu Phe Glu Phe Tyr Lys Leu Leu Gly Ser Ala
            420                 425                 430
Asp Leu Val Pro Arg Gly Ser Ala Asn Ser Ser Ser Val Asp Lys Leu
        435                 440                 445
Trp Ser His Pro Gln Phe Glu Lys Leu Glu His His His His His His
    450                 455                 460

<210> 4
<211> 464
<212> PRT
<213> Artificial Sequence

<223> "BoNT/A-Lc_ M106I, M253V, M411K including His-tag"

<220>
<223> BoNT/A-Lc_ M106I, M253V, M411K including His-tag

<400> 4
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Ile Leu Leu Thr Ser Ile Val

100                 105                 110
Arg Gly Ile Pro Phe Trp Gly Gly Ser Thr Ile Asp Thr Glu Leu Lys
        115                 120                 125
Val Ile Asp Thr Asn Cys Ile Asn Val Ile Gln Pro Asp Gly Ser Tyr
    130                 135                 140
Arg Ser Glu Glu Leu Asn Leu Val Ile Ile Gly Pro Ser Ala Asp Ile
145                 150                 155                 160
Ile Gln Phe Glu Cys Lys Ser Phe Gly His Glu Val Leu Asn Leu Thr
                165                 170                 175
Arg Asn Gly Tyr Gly Ser Thr Gln Tyr Ile Arg Phe Ser Pro Asp Phe
            180                 185                 190
Thr Phe Gly Phe Glu Glu Ser Leu Glu Val Asp Thr Asn Pro Leu Leu
        195                 200                 205
Gly Ala Gly Lys Phe Ala Thr Asp Pro Ala Val Thr Leu Ala His Glu
    210                 215                 220
Leu Ile His Ala Gly His Arg Leu Tyr Gly Ile Ala Ile Asn Pro Asn
225                 230                 235                 240
Arg Val Phe Lys Val Asn Thr Asn Ala Tyr Tyr Glu Val Ser Gly Leu
                245                 250                 255
Glu Val Ser Phe Glu Glu Leu Arg Thr Phe Gly Gly His Asp Ala Lys
            260                 265                 270
Phe Ile Asp Ser Leu Gln Glu Asn Glu Phe Arg Leu Tyr Tyr Tyr Asn
        275                 280                 285
Lys Phe Lys Asp Ile Ala Ser Thr Leu Asn Lys Ala Lys Ser Ile Val
    290                 295                 300
Gly Thr Thr Ala Ser Leu Gln Tyr Met Lys Asn Val Phe Lys Glu Lys
305                 310                 315                 320
Tyr Leu Leu Ser Glu Asp Thr Ser Gly Lys Phe Ser Val Asp Lys Leu
                325                 330                 335
Lys Phe Asp Lys Leu Tyr Lys Met Leu Thr Glu Ile Tyr Thr Glu Asp
            340                 345                 350
Asn Phe Val Lys Phe Phe Lys Val Leu Asn Arg Lys Thr Tyr Leu Asn
        355                 360                 365
Phe Asp Lys Ala Val Phe Lys Ile Asn Ile Val Pro Lys Val Asn Tyr
    370                 375                 380
Thr Ile Tyr Asp Gly Phe Asn Leu Arg Asn Thr Asn Leu Ala Ala Asn
385                 390                 395                 400
Phe Asn Gly Gln Asn Thr Glu Ile Asn Asn Lys Asn Phe Thr Lys Leu
                405                 410                 415
Lys Asn Phe Thr Gly Leu Phe Glu Phe Tyr Lys Leu Leu Gly Ser Ala
            420                 425                 430
Asp Leu Val Pro Arg Gly Ser Ala Asn Ser Ser Ser Val Asp Lys Leu
        435                 440                 445
Trp Ser His Pro Gln Phe Glu Lys Leu Glu His His His His His His
    450                 455                 460

<210> 5
<211> 464
<212> PRT
<213> Artificial Sequence

<223> "BoNT/A-Lc_ M106L/M253L/M411Q including His-tag"

<220>
<223> BoNT/A-Lc_ M106L/M253L/M411Q including His-tag

<400> 5
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30

Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Leu Leu Leu Thr Ser Ile Val
            100                 105                 110
Arg Gly Ile Pro Phe Trp Gly Gly Ser Thr Ile Asp Thr Glu Leu Lys
        115                 120                 125
Val Ile Asp Thr Asn Cys Ile Asn Val Ile Gln Pro Asp Gly Ser Tyr
    130                 135                 140
Arg Ser Glu Glu Leu Asn Leu Val Ile Ile Gly Pro Ser Ala Asp Ile
145                 150                 155                 160
Ile Gln Phe Glu Cys Lys Ser Phe Gly His Glu Val Leu Asn Leu Thr
                165                 170                 175
Arg Asn Gly Tyr Gly Ser Thr Gln Tyr Ile Arg Phe Ser Pro Asp Phe
            180                 185                 190
Thr Phe Gly Phe Glu Glu Ser Leu Glu Val Asp Thr Asn Pro Leu Leu
        195                 200                 205
Gly Ala Gly Lys Phe Ala Thr Asp Pro Ala Val Thr Leu Ala His Glu
    210                 215                 220
Leu Ile His Ala Gly His Arg Leu Tyr Gly Ile Ala Ile Asn Pro Asn
225                 230                 235                 240
Arg Val Phe Lys Val Asn Thr Asn Ala Tyr Tyr Glu Leu Ser Gly Leu
                245                 250                 255
Glu Val Ser Phe Glu Glu Leu Arg Thr Phe Gly Gly His Asp Ala Lys
            260                 265                 270
Phe Ile Asp Ser Leu Gln Glu Asn Glu Phe Arg Leu Tyr Tyr Tyr Asn
        275                 280                 285
Lys Phe Lys Asp Ile Ala Ser Thr Leu Asn Lys Ala Lys Ser Ile Val
    290                 295                 300
Gly Thr Thr Ala Ser Leu Gln Tyr Met Lys Asn Val Phe Lys Glu Lys
305                 310                 315                 320
Tyr Leu Leu Ser Glu Asp Thr Ser Gly Lys Phe Ser Val Asp Lys Leu
                325                 330                 335
Lys Phe Asp Lys Leu Tyr Lys Met Leu Thr Glu Ile Tyr Thr Glu Asp
            340                 345                 350
Asn Phe Val Lys Phe Phe Lys Val Leu Asn Arg Lys Thr Tyr Leu Asn
        355                 360                 365
Phe Asp Lys Ala Val Phe Lys Ile Asn Ile Val Pro Lys Val Asn Tyr
    370                 375                 380
Thr Ile Tyr Asp Gly Phe Asn Leu Arg Asn Thr Asn Leu Ala Ala Asn
385                 390                 395                 400
Phe Asn Gly Gln Asn Thr Glu Ile Asn Asn Gln Asn Phe Thr Lys Leu
                405                 410                 415
Lys Asn Phe Thr Gly Leu Phe Glu Phe Tyr Lys Leu Leu Gly Ser Ala
            420                 425                 430
Asp Leu Val Pro Arg Gly Ser Ala Asn Ser Ser Ser Val Asp Lys Leu
        435                 440                 445
Trp Ser His Pro Gln Phe Glu Lys Leu Glu His His His His His His
    450                 455                 460

<210> 6
<211> 464
<212> PRT
<213> Artificial Sequence

<223> "BoNT/A-Lc_ M106L/M253Y/M411K including His-tag"

<220>
<223> BoNT/A-Lc_ M106L/M253Y/M411K including His-tag

<400> 6
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Leu Leu Leu Thr Ser Ile Val
            100                 105                 110
Arg Gly Ile Pro Phe Trp Gly Gly Ser Thr Ile Asp Thr Glu Leu Lys
        115                 120                 125
Val Ile Asp Thr Asn Cys Ile Asn Val Ile Gln Pro Asp Gly Ser Tyr
    130                 135                 140
Arg Ser Glu Glu Leu Asn Leu Val Ile Ile Gly Pro Ser Ala Asp Ile
145                 150                 155                 160
Ile Gln Phe Glu Cys Lys Ser Phe Gly His Glu Val Leu Asn Leu Thr
                165                 170                 175
Arg Asn Gly Tyr Gly Ser Thr Gln Tyr Ile Arg Phe Ser Pro Asp Phe
            180                 185                 190
Thr Phe Gly Phe Glu Glu Ser Leu Glu Val Asp Thr Asn Pro Leu Leu
        195                 200                 205
Gly Ala Gly Lys Phe Ala Thr Asp Pro Ala Val Thr Leu Ala His Glu
    210                 215                 220
Leu Ile His Ala Gly His Arg Leu Tyr Gly Ile Ala Ile Asn Pro Asn
225                 230                 235                 240
Arg Val Phe Lys Val Asn Thr Asn Ala Tyr Tyr Glu Tyr Ser Gly Leu
                245                 250                 255
Glu Val Ser Phe Glu Glu Leu Arg Thr Phe Gly Gly His Asp Ala Lys
            260                 265                 270
Phe Ile Asp Ser Leu Gln Glu Asn Glu Phe Arg Leu Tyr Tyr Tyr Asn
        275                 280                 285
Lys Phe Lys Asp Ile Ala Ser Thr Leu Asn Lys Ala Lys Ser Ile Val
    290                 295                 300
Gly Thr Thr Ala Ser Leu Gln Tyr Met Lys Asn Val Phe Lys Glu Lys
305                 310                 315                 320
Tyr Leu Leu Ser Glu Asp Thr Ser Gly Lys Phe Ser Val Asp Lys Leu
                325                 330                 335
Lys Phe Asp Lys Leu Tyr Lys Met Leu Thr Glu Ile Tyr Thr Glu Asp
            340                 345                 350
Asn Phe Val Lys Phe Phe Lys Val Leu Asn Arg Lys Thr Tyr Leu Asn
        355                 360                 365
Phe Asp Lys Ala Val Phe Lys Ile Asn Ile Val Pro Lys Val Asn Tyr
    370                 375                 380
Thr Ile Tyr Asp Gly Phe Asn Leu Arg Asn Thr Asn Leu Ala Ala Asn
385                 390                 395                 400
Phe Asn Gly Gln Asn Thr Glu Ile Asn Asn Lys Asn Phe Thr Lys Leu
                405                 410                 415
Lys Asn Phe Thr Gly Leu Phe Glu Phe Tyr Lys Leu Leu Gly Ser Ala
            420                 425                 430
Asp Leu Val Pro Arg Gly Ser Ala Asn Ser Ser Ser Val Asp Lys Leu
        435                 440                 445
Trp Ser His Pro Gln Phe Glu Lys Leu Glu His His His His His His
    450                 455                 460

<210> 7
<211> 464
<212> PRT
<213> Artificial Sequence

<223> "BoNT/A-Lc_ M30F/M106L/C134I/C165I/M253L/M313I/M344L/M411Q  including
His-tag "

<220>
<223> BoNT/A-Lc_ M30F/M106L/C134I/C165I/M253L/M313I/M344L/M411Q
      including His-tag

<400> 7
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Phe Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Leu Leu Leu Thr Ser Ile Val
            100                 105                 110
Arg Gly Ile Pro Phe Trp Gly Gly Ser Thr Ile Asp Thr Glu Leu Lys
        115                 120                 125
Val Ile Asp Thr Asn Ile Ile Asn Val Ile Gln Pro Asp Gly Ser Tyr
    130                 135                 140
Arg Ser Glu Glu Leu Asn Leu Val Ile Ile Gly Pro Ser Ala Asp Ile
145                 150                 155                 160
Ile Gln Phe Glu Ile Lys Ser Phe Gly His Glu Val Leu Asn Leu Thr
                165                 170                 175
Arg Asn Gly Tyr Gly Ser Thr Gln Tyr Ile Arg Phe Ser Pro Asp Phe
            180                 185                 190
Thr Phe Gly Phe Glu Glu Ser Leu Glu Val Asp Thr Asn Pro Leu Leu
        195                 200                 205
Gly Ala Gly Lys Phe Ala Thr Asp Pro Ala Val Thr Leu Ala His Glu
    210                 215                 220
Leu Ile His Ala Gly His Arg Leu Tyr Gly Ile Ala Ile Asn Pro Asn
225                 230                 235                 240
Arg Val Phe Lys Val Asn Thr Asn Ala Tyr Tyr Glu Leu Ser Gly Leu
                245                 250                 255
Glu Val Ser Phe Glu Glu Leu Arg Thr Phe Gly Gly His Asp Ala Lys
            260                 265                 270
Phe Ile Asp Ser Leu Gln Glu Asn Glu Phe Arg Leu Tyr Tyr Tyr Asn
        275                 280                 285
Lys Phe Lys Asp Ile Ala Ser Thr Leu Asn Lys Ala Lys Ser Ile Val
    290                 295                 300
Gly Thr Thr Ala Ser Leu Gln Tyr Ile Lys Asn Val Phe Lys Glu Lys
305                 310                 315                 320
Tyr Leu Leu Ser Glu Asp Thr Ser Gly Lys Phe Ser Val Asp Lys Leu
                325                 330                 335
Lys Phe Asp Lys Leu Tyr Lys Leu Leu Thr Glu Ile Tyr Thr Glu Asp
            340                 345                 350
Asn Phe Val Lys Phe Phe Lys Val Leu Asn Arg Lys Thr Tyr Leu Asn
        355                 360                 365
Phe Asp Lys Ala Val Phe Lys Ile Asn Ile Val Pro Lys Val Asn Tyr

370                 375                 380
Thr Ile Tyr Asp Gly Phe Asn Leu Arg Asn Thr Asn Leu Ala Ala Asn
385                 390                 395                 400
Phe Asn Gly Gln Asn Thr Glu Ile Asn Asn Gln Asn Phe Thr Lys Leu
                405                 410                 415
Lys Asn Phe Thr Gly Leu Phe Glu Phe Tyr Lys Leu Leu Gly Ser Ala
            420                 425                 430
Asp Leu Val Pro Arg Gly Ser Ala Asn Ser Ser Ser Val Asp Lys Leu
        435                 440                 445
Trp Ser His Pro Gln Phe Glu Lys Leu Glu His His His His His His
    450                 455                 460

<210> 8
<211> 464
<212> PRT
<213> Artificial Sequence

<223> "BoNT/A-Lc_ M30Y/M106L/C134I/M253L/M344L/M411Q including His-tag "

<220>
<223> BoNT/A-Lc_ M30Y/M106L/C134I/M253L/M344L/M411Q including His-tag

<400> 8
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Tyr Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Leu Leu Leu Thr Ser Ile Val
            100                 105                 110
Arg Gly Ile Pro Phe Trp Gly Gly Ser Thr Ile Asp Thr Glu Leu Lys
        115                 120                 125
Val Ile Asp Thr Asn Ile Ile Asn Val Ile Gln Pro Asp Gly Ser Tyr
    130                 135                 140
Arg Ser Glu Glu Leu Asn Leu Val Ile Ile Gly Pro Ser Ala Asp Ile
145                 150                 155                 160
Ile Gln Phe Glu Cys Lys Ser Phe Gly His Glu Val Leu Asn Leu Thr
                165                 170                 175
Arg Asn Gly Tyr Gly Ser Thr Gln Tyr Ile Arg Phe Ser Pro Asp Phe
            180                 185                 190
Thr Phe Gly Phe Glu Glu Ser Leu Glu Val Asp Thr Asn Pro Leu Leu
        195                 200                 205
Gly Ala Gly Lys Phe Ala Thr Asp Pro Ala Val Thr Leu Ala His Glu
    210                 215                 220
Leu Ile His Ala Gly His Arg Leu Tyr Gly Ile Ala Ile Asn Pro Asn
225                 230                 235                 240
Arg Val Phe Lys Val Asn Thr Asn Ala Tyr Tyr Glu Leu Ser Gly Leu
                245                 250                 255
Glu Val Ser Phe Glu Glu Leu Arg Thr Phe Gly Gly His Asp Ala Lys
            260                 265                 270
Phe Ile Asp Ser Leu Gln Glu Asn Glu Phe Arg Leu Tyr Tyr Tyr Asn
        275                 280                 285
Lys Phe Lys Asp Ile Ala Ser Thr Leu Asn Lys Ala Lys Ser Ile Val
    290                 295                 300

Gly Thr Thr Ala Ser Leu Gln Tyr Ile Lys Asn Val Phe Lys Glu Lys
305                 310                 315                 320
Tyr Leu Leu Ser Glu Asp Thr Ser Gly Lys Phe Ser Val Asp Lys Leu
                325                 330                 335
Lys Phe Asp Lys Leu Tyr Lys Leu Leu Thr Glu Ile Tyr Thr Glu Asp
            340                 345                 350
Asn Phe Val Lys Phe Phe Lys Val Leu Asn Arg Lys Thr Tyr Leu Asn
        355                 360                 365
Phe Asp Lys Ala Val Phe Lys Ile Asn Ile Val Pro Lys Val Asn Tyr
    370                 375                 380
Thr Ile Tyr Asp Gly Phe Asn Leu Arg Asn Thr Asn Leu Ala Ala Asn
385                 390                 395                 400
Phe Asn Gly Gln Asn Thr Glu Ile Asn Asn Gln Asn Phe Thr Lys Leu
                405                 410                 415
Lys Asn Phe Thr Gly Leu Phe Glu Phe Tyr Lys Leu Leu Gly Ser Ala
            420                 425                 430
Asp Leu Val Pro Arg Gly Ser Ala Asn Ser Ser Ser Val Asp Lys Leu
        435                 440                 445
Trp Ser His Pro Gln Phe Glu Lys Leu Glu His His His His His His
    450                 455                 460

<210> 9
<211> 462
<212> PRT
<213> Artificial Sequence

<223> "BoNT/A-Lc_ 2627 including His-tag"

<220>
<223> BoNT/A-Lc_ 2627 including His-tag

<400> 9
Met Pro Phe Val Asn Lys Gln Phe Asn Tyr Lys Asp Pro Val Asn Gly
1               5                   10                  15
Val Asp Ile Ala Tyr Ile Lys Ile Pro Gly Gln Met Gln Pro Val Lys
            20                  25                  30
Ala Phe Lys Ile His Asn Lys Ile Trp Val Ile Pro Glu Arg Asp Thr
        35                  40                  45
Phe Thr Asn Pro Glu Glu Gly Asp Leu Asn Pro Pro Pro Glu Ala Lys
    50                  55                  60
Gln Val Pro Val Ser Tyr Tyr Asp Ser Thr Tyr Leu Ser Thr Asp Asn
65                  70                  75                  80
Glu Lys Asp Asn Tyr Leu Lys Gly Val Thr Lys Leu Phe Glu Arg Ile
                85                  90                  95
Tyr Ser Thr Asp Leu Gly Arg Met Leu Leu Thr Ser Ile Val Arg Gly
            100                 105                 110
Ile Pro Phe Trp Gly Gly Ser Thr Ile Asp Thr Glu Leu Lys Val Ile
        115                 120                 125
Asp Thr Asn Cys Ile Asn Val Ile Gln Pro Asp Gly Ser Tyr Arg Ser
    130                 135                 140
Glu Glu Leu Asn Leu Val Ile Ile Gly Pro Ser Ala Asp Ile Ile Gln
145                 150                 155                 160
Phe Glu Cys Lys Ser Phe Gly His Glu Val Leu Asn Leu Thr Arg Asn
                165                 170                 175
Gly Tyr Gly Ser Thr Gln Tyr Ile Arg Phe Ser Pro Asp Phe Thr Phe
            180                 185                 190
Gly Phe Glu Glu Ser Leu Glu Val Asp Thr Asn Pro Leu Leu Gly Ala
        195                 200                 205
Gly Lys Phe Ala Thr Asp Pro Ala Val Thr Leu Ala His Glu Leu Ile
    210                 215                 220
His Ala Gly His Arg Leu Tyr Gly Ile Ala Ile Asn Pro Asn Arg Val

225                 230                 235                 240
Phe Lys Val Asn Thr Asn Ala Tyr Tyr Glu Met Ser Gly Leu Glu Val
                245                 250                 255
Ser Phe Glu Glu Leu Arg Thr Phe Gly Gly His Asp Ala Lys Phe Ile
            260                 265                 270
Asp Ser Leu Gln Glu Asn Glu Phe Arg Leu Tyr Tyr Tyr Asn Lys Phe
        275                 280                 285
Lys Asp Ile Ala Ser Thr Leu Asn Lys Ala Lys Ser Ile Val Gly Thr
    290                 295                 300
Thr Ala Ser Leu Gln Tyr Met Lys Asn Val Phe Lys Glu Lys Tyr Leu
305                 310                 315                 320
Leu Ser Glu Asp Thr Ser Gly Lys Phe Ser Val Asp Lys Leu Lys Phe
                325                 330                 335
Asp Lys Leu Tyr Lys Met Leu Thr Glu Ile Tyr Thr Glu Asp Asn Phe
            340                 345                 350
Val Lys Phe Phe Lys Val Leu Asn Arg Lys Thr Tyr Leu Asn Phe Asp
        355                 360                 365
Lys Ala Val Phe Lys Ile Asn Ile Val Pro Lys Val Asn Tyr Thr Ile
    370                 375                 380
Tyr Asp Gly Phe Asn Leu Arg Asn Thr Asn Leu Ala Ala Asn Phe Asn
385                 390                 395                 400
Gly Gln Asn Thr Glu Ile Asn Asn Met Asn Phe Thr Lys Leu Lys Asn
                405                 410                 415
Phe Thr Gly Leu Phe Glu Phe Tyr Lys Leu Leu Gly Ser Ala Asp Leu
            420                 425                 430
Val Pro Arg Gly Ser Ala Asn Ser Ser Ser Val Asp Lys Leu Trp Ser
        435                 440                 445
His Pro Gln Phe Glu Lys Leu Glu His His His His His His
    450                 455                 460

<210> 10
<211> 462
<212> PRT
<213> Artificial Sequence

<223> "BoNT/A-Lc_ 6465 including His-tag"

<220>
<223> BoNT/A-Lc_ 6465 including His-tag

<400> 10
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Lys
    50                  55                  60
Gln Val Pro Val Ser Tyr Tyr Asp Ser Thr Tyr Leu Ser Thr Asp Asn
65                  70                  75                  80
Glu Lys Asp Asn Tyr Leu Lys Gly Val Thr Lys Leu Phe Glu Arg Ile
                85                  90                  95
Tyr Ser Thr Asp Leu Gly Arg Met Leu Leu Thr Ser Ile Val Arg Gly
            100                 105                 110
Ile Pro Phe Trp Gly Gly Ser Thr Ile Asp Thr Glu Leu Lys Val Ile
        115                 120                 125
Asp Thr Asn Cys Ile Asn Val Ile Gln Pro Asp Gly Ser Tyr Arg Ser
    130                 135                 140
Glu Glu Leu Asn Leu Val Ile Ile Gly Pro Ser Ala Asp Ile Ile Gln
145                 150                 155                 160
Phe Glu Cys Lys Ser Phe Gly His Glu Val Leu Asn Leu Thr Arg Asn
                165                 170                 175

Gly Tyr Gly Ser Thr Gln Tyr Ile Arg Phe Ser Pro Asp Phe Thr Phe
            180                 185                 190
Gly Phe Glu Glu Ser Leu Glu Val Asp Thr Asn Pro Leu Leu Gly Ala
        195                 200                 205
Gly Lys Phe Ala Thr Asp Pro Ala Val Thr Leu Ala His Glu Leu Ile
    210                 215                 220
His Ala Gly His Arg Leu Tyr Gly Ile Ala Ile Asn Pro Asn Arg Val
225                 230                 235                 240
Phe Lys Val Asn Thr Asn Ala Tyr Tyr Glu Met Ser Gly Leu Glu Val
                245                 250                 255
Ser Phe Glu Glu Leu Arg Thr Phe Gly Gly His Asp Ala Lys Phe Ile
            260                 265                 270
Asp Ser Leu Gln Glu Asn Glu Phe Arg Leu Tyr Tyr Tyr Asn Lys Phe
        275                 280                 285
Lys Asp Ile Ala Ser Thr Leu Asn Lys Ala Lys Ser Ile Val Gly Thr
    290                 295                 300
Thr Ala Ser Leu Gln Tyr Met Lys Asn Val Phe Lys Glu Lys Tyr Leu
305                 310                 315                 320
Leu Ser Glu Asp Thr Ser Gly Lys Phe Ser Val Asp Lys Leu Lys Phe
                325                 330                 335
Asp Lys Leu Tyr Lys Met Leu Thr Glu Ile Tyr Thr Glu Asp Asn Phe
            340                 345                 350
Val Lys Phe Phe Lys Val Leu Asn Arg Lys Thr Tyr Leu Asn Phe Asp
        355                 360                 365
Lys Ala Val Phe Lys Ile Asn Ile Val Pro Lys Val Asn Tyr Thr Ile
    370                 375                 380
Tyr Asp Gly Phe Asn Leu Arg Asn Thr Asn Leu Ala Ala Asn Phe Asn
385                 390                 395                 400
Gly Gln Asn Thr Glu Ile Asn Asn Met Asn Phe Thr Lys Leu Lys Asn
                405                 410                 415
Phe Thr Gly Leu Phe Glu Phe Tyr Lys Leu Leu Gly Ser Ala Asp Leu
            420                 425                 430
Val Pro Arg Gly Ser Ala Asn Ser Ser Ser Val Asp Lys Leu Trp Ser
        435                 440                 445
His Pro Gln Phe Glu Lys Leu Glu His His His His His His
    450                 455                 460

<210> 11
<211> 463
<212> PRT
<213> Artificial Sequence

<223> "BoNT/A-Lc_ 125 including His-tag"

<220>
<223> BoNT/A-Lc_ 125 including His-tag

<400> 11
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Met Leu Leu Thr Ser Ile Val
            100                 105                 110
Arg Gly Ile Pro Phe Trp Gly Gly Ser Thr Ile Asp Glu Leu Lys Val

115                 120                 125
Ile Asp Thr Asn Cys Ile Asn Val Ile Gln Pro Asp Gly Ser Tyr Arg
    130                 135                 140
Ser Glu Glu Leu Asn Leu Val Ile Ile Gly Pro Ser Ala Asp Ile Ile
145                 150                 155                 160
Gln Phe Glu Cys Lys Ser Phe Gly His Glu Val Leu Asn Leu Thr Arg
                165                 170                 175
Asn Gly Tyr Gly Ser Thr Gln Tyr Ile Arg Phe Ser Pro Asp Phe Thr
            180                 185                 190
Phe Gly Phe Glu Glu Ser Leu Glu Val Asp Thr Asn Pro Leu Leu Gly
        195                 200                 205
Ala Gly Lys Phe Ala Thr Asp Pro Ala Val Thr Leu Ala His Glu Leu
    210                 215                 220
Ile His Ala Gly His Arg Leu Tyr Gly Ile Ala Ile Asn Pro Asn Arg
225                 230                 235                 240
Val Phe Lys Val Asn Thr Asn Ala Tyr Tyr Glu Met Ser Gly Leu Glu
                245                 250                 255
Val Ser Phe Glu Glu Leu Arg Thr Phe Gly Gly His Asp Ala Lys Phe
            260                 265                 270
Ile Asp Ser Leu Gln Glu Asn Glu Phe Arg Leu Tyr Tyr Tyr Asn Lys
        275                 280                 285
Phe Lys Asp Ile Ala Ser Thr Leu Asn Lys Ala Lys Ser Ile Val Gly
    290                 295                 300
Thr Thr Ala Ser Leu Gln Tyr Met Lys Asn Val Phe Lys Glu Lys Tyr
305                 310                 315                 320
Leu Leu Ser Glu Asp Thr Ser Gly Lys Phe Ser Val Asp Lys Leu Lys
                325                 330                 335
Phe Asp Lys Leu Tyr Lys Met Leu Thr Glu Ile Tyr Thr Glu Asp Asn
            340                 345                 350
Phe Val Lys Phe Phe Lys Val Leu Asn Arg Lys Thr Tyr Leu Asn Phe
        355                 360                 365
Asp Lys Ala Val Phe Lys Ile Asn Ile Val Pro Lys Val Asn Tyr Thr
    370                 375                 380
Ile Tyr Asp Gly Phe Asn Leu Arg Asn Thr Asn Leu Ala Ala Asn Phe
385                 390                 395                 400
Asn Gly Gln Asn Thr Glu Ile Asn Asn Met Asn Phe Thr Lys Leu Lys
                405                 410                 415
Asn Phe Thr Gly Leu Phe Glu Phe Tyr Lys Leu Leu Gly Ser Ala Asp
            420                 425                 430
Leu Val Pro Arg Gly Ser Ala Asn Ser Ser Ser Val Asp Lys Leu Trp
        435                 440                 445
Ser His Pro Gln Phe Glu Lys Leu Glu His His His His His His
    450                 455                 460

<210> 12
<211> 463
<212> PRT
<213> Artificial Sequence

<223> "BoNT/A-Lc_ 122 including His-tag"

<220>
<223> BoNT/A-Lc_ 122 including His-tag

<400> 12
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60

Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Met Leu Leu Thr Ser Ile Val
            100                 105                 110
Arg Gly Ile Pro Phe Trp Gly Gly Ser Ile Asp Thr Glu Leu Lys Val
        115                 120                 125
Ile Asp Thr Asn Cys Ile Asn Val Ile Gln Pro Asp Gly Ser Tyr Arg
    130                 135                 140
Ser Glu Glu Leu Asn Leu Val Ile Ile Gly Pro Ser Ala Asp Ile Ile
145                 150                 155                 160
Gln Phe Glu Cys Lys Ser Phe Gly His Glu Val Leu Asn Leu Thr Arg
                165                 170                 175
Asn Gly Tyr Gly Ser Thr Gln Tyr Ile Arg Phe Ser Pro Asp Phe Thr
            180                 185                 190
Phe Gly Phe Glu Glu Ser Leu Glu Val Asp Thr Asn Pro Leu Leu Gly
        195                 200                 205
Ala Gly Lys Phe Ala Thr Asp Pro Ala Val Thr Leu Ala His Glu Leu
    210                 215                 220
Ile His Ala Gly His Arg Leu Tyr Gly Ile Ala Ile Asn Pro Asn Arg
225                 230                 235                 240
Val Phe Lys Val Asn Thr Asn Ala Tyr Tyr Glu Met Ser Gly Leu Glu
                245                 250                 255
Val Ser Phe Glu Glu Leu Arg Thr Phe Gly Gly His Asp Ala Lys Phe
            260                 265                 270
Ile Asp Ser Leu Gln Glu Asn Glu Phe Arg Leu Tyr Tyr Tyr Asn Lys
        275                 280                 285
Phe Lys Asp Ile Ala Ser Thr Leu Asn Lys Ala Lys Ser Ile Val Gly
    290                 295                 300
Thr Thr Ala Ser Leu Gln Tyr Met Lys Asn Val Phe Lys Glu Lys Tyr
305                 310                 315                 320
Leu Leu Ser Glu Asp Thr Ser Gly Lys Phe Ser Val Asp Lys Leu Lys
                325                 330                 335
Phe Asp Lys Leu Tyr Lys Met Leu Thr Glu Ile Tyr Thr Glu Asp Asn
            340                 345                 350
Phe Val Lys Phe Phe Lys Val Leu Asn Arg Lys Thr Tyr Leu Asn Phe
        355                 360                 365
Asp Lys Ala Val Phe Lys Ile Asn Ile Val Pro Lys Val Asn Tyr Thr
    370                 375                 380
Ile Tyr Asp Gly Phe Asn Leu Arg Asn Thr Asn Leu Ala Ala Asn Phe
385                 390                 395                 400
Asn Gly Gln Asn Thr Glu Ile Asn Asn Met Asn Phe Thr Lys Leu Lys
                405                 410                 415
Asn Phe Thr Gly Leu Phe Glu Phe Tyr Lys Leu Leu Gly Ser Ala Asp
            420                 425                 430
Leu Val Pro Arg Gly Ser Ala Asn Ser Ser Ser Val Asp Lys Leu Trp
        435                 440                 445
Ser His Pro Gln Phe Glu Lys Leu Glu His His His His His His
    450                 455                 460

<210> 13
<211> 461
<212> PRT
<213> Artificial Sequence

<223> "BoNT/A-Lc_ 6465_125 including His-tag"

<220>
<223> BoNT/A-Lc_ 6465_125 including His-tag

<400> 13
Met Pro Phe Val Asn Lys Gln Phe Asn Tyr Lys Asp Pro Val Asn Gly

1                   5                   10                  15
Val Asp Ile Ala Tyr Ile Lys Ile Pro Asn Ala Gly Gln Met Gln Pro
                20                  25                  30
Val Lys Ala Phe Lys Ile His Asn Lys Ile Trp Val Ile Pro Glu Arg
            35                  40                  45
Asp Thr Phe Thr Asn Pro Glu Glu Gly Asp Leu Asn Pro Pro Pro Lys
    50                  55                  60
Gln Val Pro Val Ser Tyr Tyr Asp Ser Thr Tyr Leu Ser Thr Asp Asn
65                  70                  75                  80
Glu Lys Asp Asn Tyr Leu Lys Gly Val Thr Lys Leu Phe Glu Arg Ile
                85                  90                  95
Tyr Ser Thr Asp Leu Gly Arg Met Leu Leu Thr Ser Ile Val Arg Gly
            100                 105                 110
Ile Pro Phe Trp Gly Gly Ser Thr Ile Asp Glu Leu Lys Val Ile Asp
        115                 120                 125
Thr Asn Cys Ile Asn Val Ile Gln Pro Asp Gly Ser Tyr Arg Ser Glu
    130                 135                 140
Glu Leu Asn Leu Val Ile Ile Gly Pro Ser Ala Asp Ile Ile Gln Phe
145                 150                 155                 160
Glu Cys Lys Ser Phe Gly His Glu Val Leu Asn Leu Thr Arg Asn Gly
                165                 170                 175
Tyr Gly Ser Thr Gln Tyr Ile Arg Phe Ser Pro Asp Phe Thr Phe Gly
            180                 185                 190
Phe Glu Glu Ser Leu Glu Val Asp Thr Asn Pro Leu Leu Gly Ala Gly
        195                 200                 205
Lys Phe Ala Thr Asp Pro Ala Val Thr Leu Ala His Glu Leu Ile His
    210                 215                 220
Ala Gly His Arg Leu Tyr Gly Ile Ala Ile Asn Pro Asn Arg Val Phe
225                 230                 235                 240
Lys Val Asn Thr Asn Ala Tyr Tyr Glu Met Ser Gly Leu Glu Val Ser
                245                 250                 255
Phe Glu Glu Leu Arg Thr Phe Gly Gly His Asp Ala Lys Phe Ile Asp
            260                 265                 270
Ser Leu Gln Glu Asn Glu Phe Arg Leu Tyr Tyr Tyr Asn Lys Phe Lys
        275                 280                 285
Asp Ile Ala Ser Thr Leu Asn Lys Ala Lys Ser Ile Val Gly Thr Thr
    290                 295                 300
Ala Ser Leu Gln Tyr Met Lys Asn Val Phe Lys Glu Lys Tyr Leu Leu
305                 310                 315                 320
Ser Glu Asp Thr Ser Gly Lys Phe Ser Val Asp Lys Leu Lys Phe Asp
                325                 330                 335
Lys Leu Tyr Lys Met Leu Thr Glu Ile Tyr Thr Glu Asp Asn Phe Val
            340                 345                 350
Lys Phe Phe Lys Val Leu Asn Arg Lys Thr Tyr Leu Asn Phe Asp Lys
        355                 360                 365
Ala Val Phe Lys Ile Asn Ile Val Pro Lys Val Asn Tyr Thr Ile Tyr
    370                 375                 380
Asp Gly Phe Asn Leu Arg Asn Thr Asn Leu Ala Ala Asn Phe Asn Gly
385                 390                 395                 400
Gln Asn Thr Glu Ile Asn Asn Met Asn Phe Thr Lys Leu Lys Asn Phe
                405                 410                 415
Thr Gly Leu Phe Glu Phe Tyr Lys Leu Leu Gly Ser Ala Asp Leu Val
            420                 425                 430
Pro Arg Gly Ser Ala Asn Ser Ser Ser Val Asp Lys Leu Trp Ser His
        435                 440                 445
Pro Gln Phe Glu Lys Leu Glu His His His His His His
    450                 455                 460

<210> 14
<211> 459
<212> PRT
<213> Artificial Sequence

<223> "BoNT/A-Lc_2627_6465_125  including His-tag"

<220>
<223> BoNT/A-Lc_2627_6465_125 including His-tag

<400> 14
Met Pro Phe Val Asn Lys Gln Phe Asn Tyr Lys Asp Pro Val Asn Gly
1               5                   10                  15
Val Asp Ile Ala Tyr Ile Lys Ile Pro Gly Gln Met Gln Pro Val Lys
            20                  25                  30
Ala Phe Lys Ile His Asn Lys Ile Trp Val Ile Pro Glu Arg Asp Thr
        35                  40                  45
Phe Thr Asn Pro Glu Glu Gly Asp Leu Asn Pro Pro Pro Lys Gln Val
    50                  55                  60
Pro Val Ser Tyr Tyr Asp Ser Thr Tyr Leu Ser Thr Asp Asn Glu Lys
65                  70                  75                  80
Asp Asn Tyr Leu Lys Gly Val Thr Lys Leu Phe Glu Arg Ile Tyr Ser
                85                  90                  95
Thr Asp Leu Gly Arg Met Leu Leu Thr Ser Ile Val Arg Gly Ile Pro
            100                 105                 110
Phe Trp Gly Gly Ser Thr Ile Asp Glu Leu Lys Val Ile Asp Thr Asn
        115                 120                 125
Cys Ile Asn Val Ile Gln Pro Asp Gly Ser Tyr Arg Ser Glu Glu Leu
    130                 135                 140
Asn Leu Val Ile Ile Gly Pro Ser Ala Asp Ile Ile Gln Phe Glu Cys
145                 150                 155                 160
Lys Ser Phe Gly His Glu Val Leu Asn Leu Thr Arg Asn Gly Tyr Gly
                165                 170                 175
Ser Thr Gln Tyr Ile Arg Phe Ser Pro Asp Phe Thr Phe Gly Phe Glu
            180                 185                 190
Glu Ser Leu Glu Val Asp Thr Asn Pro Leu Leu Gly Ala Gly Lys Phe
        195                 200                 205
Ala Thr Asp Pro Ala Val Thr Leu Ala His Glu Leu Ile His Ala Gly
    210                 215                 220
His Arg Leu Tyr Gly Ile Ala Ile Asn Pro Asn Arg Val Phe Lys Val
225                 230                 235                 240
Asn Thr Asn Ala Tyr Tyr Glu Met Ser Gly Leu Glu Val Ser Phe Glu
                245                 250                 255
Glu Leu Arg Thr Phe Gly Gly His Asp Ala Lys Phe Ile Asp Ser Leu
            260                 265                 270
Gln Glu Asn Glu Phe Arg Leu Tyr Tyr Tyr Asn Lys Phe Lys Asp Ile
        275                 280                 285
Ala Ser Thr Leu Asn Lys Ala Lys Ser Ile Val Gly Thr Thr Ala Ser
    290                 295                 300
Leu Gln Tyr Met Lys Asn Val Phe Lys Glu Lys Tyr Leu Leu Ser Glu
305                 310                 315                 320
Asp Thr Ser Gly Lys Phe Ser Val Asp Lys Leu Lys Phe Asp Lys Leu
                325                 330                 335
Tyr Lys Met Leu Thr Glu Ile Tyr Thr Glu Asp Asn Phe Val Lys Phe
            340                 345                 350
Phe Lys Val Leu Asn Arg Lys Thr Tyr Leu Asn Phe Asp Lys Ala Val
        355                 360                 365
Phe Lys Ile Asn Ile Val Pro Lys Val Asn Tyr Thr Ile Tyr Asp Gly
    370                 375                 380
Phe Asn Leu Arg Asn Thr Asn Leu Ala Ala Asn Phe Asn Gly Gln Asn
385                 390                 395                 400
Thr Glu Ile Asn Asn Met Asn Phe Thr Lys Leu Lys Asn Phe Thr Gly
                405                 410                 415
Leu Phe Glu Phe Tyr Lys Leu Leu Gly Ser Ala Asp Leu Val Pro Arg
            420                 425                 430
Gly Ser Ala Asn Ser Ser Ser Val Asp Lys Leu Trp Ser His Pro Gln
        435                 440                 445
Phe Glu Lys Leu Glu His His His His His His

450                 455

<210> 15
<211> 461
<212> PRT
<213> Artificial Sequence

<223> "BoNT/A-Lc__6465_122 including His-tag"

<220>
<223> BoNT/A-Lc__6465_122 including His-tag

<400> 15
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Lys
    50                  55                  60
Gln Val Pro Val Ser Tyr Tyr Asp Ser Thr Tyr Leu Ser Thr Asp Asn
65                  70                  75                  80
Glu Lys Asp Asn Tyr Leu Lys Gly Val Thr Lys Leu Phe Glu Arg Ile
                85                  90                  95
Tyr Ser Thr Asp Leu Gly Arg Met Leu Leu Thr Ser Ile Val Arg Gly
            100                 105                 110
Ile Pro Phe Trp Gly Gly Ser Ile Asp Thr Glu Leu Lys Val Ile Asp
        115                 120                 125
Thr Asn Cys Ile Asn Val Ile Gln Pro Asp Gly Ser Tyr Arg Ser Glu
    130                 135                 140
Glu Leu Asn Leu Val Ile Ile Gly Pro Ser Ala Asp Ile Ile Gln Phe
145                 150                 155                 160
Glu Cys Lys Ser Phe Gly His Glu Val Leu Asn Leu Thr Arg Asn Gly
                165                 170                 175
Tyr Gly Ser Thr Gln Tyr Ile Arg Phe Ser Pro Asp Phe Thr Phe Gly
            180                 185                 190
Phe Glu Glu Ser Leu Glu Val Asp Thr Asn Pro Leu Leu Gly Ala Gly
        195                 200                 205
Lys Phe Ala Thr Asp Pro Ala Val Thr Leu Ala His Glu Leu Ile His
    210                 215                 220
Ala Gly His Arg Leu Tyr Gly Ile Ala Ile Asn Pro Asn Arg Val Phe
225                 230                 235                 240
Lys Val Asn Thr Asn Ala Tyr Tyr Glu Met Ser Gly Leu Glu Val Ser
                245                 250                 255
Phe Glu Glu Leu Arg Thr Phe Gly Gly His Asp Ala Lys Phe Ile Asp
            260                 265                 270
Ser Leu Gln Glu Asn Glu Phe Arg Leu Tyr Tyr Tyr Asn Lys Phe Lys
        275                 280                 285
Asp Ile Ala Ser Thr Leu Asn Lys Ala Lys Ser Ile Val Gly Thr Thr
    290                 295                 300
Ala Ser Leu Gln Tyr Met Lys Asn Val Phe Lys Glu Lys Tyr Leu Leu
305                 310                 315                 320
Ser Glu Asp Thr Ser Gly Lys Phe Ser Val Asp Lys Leu Lys Phe Asp
                325                 330                 335
Lys Leu Tyr Lys Met Leu Thr Glu Ile Tyr Thr Glu Asp Asn Phe Val
            340                 345                 350
Lys Phe Phe Lys Val Leu Asn Arg Lys Thr Tyr Leu Asn Phe Asp Lys
        355                 360                 365
Ala Val Phe Lys Ile Asn Ile Val Pro Lys Val Asn Tyr Thr Ile Tyr
    370                 375                 380
Asp Gly Phe Asn Leu Arg Asn Thr Asn Leu Ala Ala Asn Phe Asn Gly
385                 390                 395                 400

Gln Asn Thr Glu Ile Asn Asn Met Asn Phe Thr Lys Leu Lys Asn Phe
                405                 410                 415
Thr Gly Leu Phe Glu Phe Tyr Lys Leu Leu Gly Ser Ala Asp Leu Val
            420                 425                 430
Pro Arg Gly Ser Ala Asn Ser Ser Ser Val Asp Lys Leu Trp Ser His
        435                 440                 445
Pro Gln Phe Glu Lys Leu Glu His His His His His His
    450                 455                 460

<210> 16
<211> 461
<212> PRT
<213> Artificial Sequence

<223> "BoNT/A-Lc__2627_122 including His-tag   "

<220>
<223> BoNT/A-Lc__2627_122 including His-tag

<400> 16
Met Pro Phe Val Asn Lys Gln Phe Asn Tyr Lys Asp Pro Val Asn Gly
1               5                   10                  15
Val Asp Ile Ala Tyr Ile Lys Ile Pro Gly Gln Met Gln Pro Val Lys
            20                  25                  30
Ala Phe Lys Ile His Asn Lys Ile Trp Val Ile Pro Glu Arg Asp Thr
        35                  40                  45
Phe Thr Asn Pro Glu Glu Gly Asp Leu Asn Pro Pro Pro Glu Ala Lys
    50                  55                  60
Gln Val Pro Val Ser Tyr Tyr Asp Ser Thr Tyr Leu Ser Thr Asp Asn
65                  70                  75                  80
Glu Lys Asp Asn Tyr Leu Lys Gly Val Thr Lys Leu Phe Glu Arg Ile
                85                  90                  95
Tyr Ser Thr Asp Leu Gly Arg Met Leu Leu Thr Ser Ile Val Arg Gly
            100                 105                 110
Ile Pro Phe Trp Gly Gly Ser Ile Asp Thr Glu Leu Lys Val Ile Asp
        115                 120                 125
Thr Asn Cys Ile Asn Val Ile Gln Pro Asp Gly Ser Tyr Arg Ser Glu
    130                 135                 140
Glu Leu Asn Leu Val Ile Ile Gly Pro Ser Ala Asp Ile Ile Gln Phe
145                 150                 155                 160
Glu Cys Lys Ser Phe Gly His Glu Val Leu Asn Leu Thr Arg Asn Gly
                165                 170                 175
Tyr Gly Ser Thr Gln Tyr Ile Arg Phe Ser Pro Asp Phe Thr Phe Gly
            180                 185                 190
Phe Glu Glu Ser Leu Glu Val Asp Thr Asn Pro Leu Leu Gly Ala Gly
        195                 200                 205
Lys Phe Ala Thr Asp Pro Ala Val Thr Leu Ala His Glu Leu Ile His
    210                 215                 220
Ala Gly His Arg Leu Tyr Gly Ile Ala Ile Asn Pro Asn Arg Val Phe
225                 230                 235                 240
Lys Val Asn Thr Asn Ala Tyr Tyr Glu Met Ser Gly Leu Glu Val Ser
                245                 250                 255
Phe Glu Glu Leu Arg Thr Phe Gly Gly His Asp Ala Lys Phe Ile Asp
            260                 265                 270
Ser Leu Gln Glu Asn Glu Phe Arg Leu Tyr Tyr Tyr Asn Lys Phe Lys
        275                 280                 285
Asp Ile Ala Ser Thr Leu Asn Lys Ala Lys Ser Ile Val Gly Thr Thr
    290                 295                 300
Ala Ser Leu Gln Tyr Met Lys Asn Val Phe Lys Glu Lys Tyr Leu Leu
305                 310                 315                 320
Ser Glu Asp Thr Ser Gly Lys Phe Ser Val Asp Lys Leu Lys Phe Asp
                325                 330                 335
Lys Leu Tyr Lys Met Leu Thr Glu Ile Tyr Thr Glu Asp Asn Phe Val

340                 345                 350
Lys Phe Phe Lys Val Leu Asn Arg Lys Thr Tyr Leu Asn Phe Asp Lys
        355                 360                 365
Ala Val Phe Lys Ile Asn Ile Val Pro Lys Val Asn Tyr Thr Ile Tyr
    370                 375                 380
Asp Gly Phe Asn Leu Arg Asn Thr Asn Leu Ala Ala Asn Phe Asn Gly
385                 390                 395                 400
Gln Asn Thr Glu Ile Asn Asn Met Asn Phe Thr Lys Leu Lys Asn Phe
                405                 410                 415
Thr Gly Leu Phe Glu Phe Tyr Lys Leu Leu Gly Ser Ala Asp Leu Val
            420                 425                 430
Pro Arg Gly Ser Ala Asn Ser Ser Ser Val Asp Lys Leu Trp Ser His
        435                 440                 445
Pro Gln Phe Glu Lys Leu Glu His His His His His His
    450                 455                 460

<210> 17
<211> 462
<212> PRT
<213> Artificial Sequence

<223> "BoNT/A-Lc_ 6465_I138W/Y144W including His-tag"

<220>
<223> BoNT/A-Lc_ 6465_I138W/Y144W including His-tag

<400> 17
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Lys
    50                  55                  60
Gln Val Pro Val Ser Tyr Tyr Asp Ser Thr Tyr Leu Ser Thr Asp Asn
65                  70                  75                  80
Glu Lys Asp Asn Tyr Leu Lys Gly Val Thr Lys Leu Phe Glu Arg Ile
                85                  90                  95
Tyr Ser Thr Asp Leu Gly Arg Met Leu Leu Thr Ser Ile Val Arg Gly
            100                 105                 110
Ile Pro Phe Trp Gly Gly Ser Thr Ile Asp Thr Glu Leu Lys Val Ile
        115                 120                 125
Asp Thr Asn Cys Ile Asn Val Trp Gln Pro Asp Gly Ser Trp Arg Ser
    130                 135                 140
Glu Glu Leu Asn Leu Val Ile Ile Gly Pro Ser Ala Asp Ile Ile Gln
145                 150                 155                 160
Phe Glu Cys Lys Ser Phe Gly His Glu Val Leu Asn Leu Thr Arg Asn
                165                 170                 175
Gly Tyr Gly Ser Thr Gln Tyr Ile Arg Phe Ser Pro Asp Phe Thr Phe
            180                 185                 190
Gly Phe Glu Glu Ser Leu Glu Val Asp Thr Asn Pro Leu Leu Gly Ala
        195                 200                 205
Gly Lys Phe Ala Thr Asp Pro Ala Val Thr Leu Ala His Glu Leu Ile
    210                 215                 220
His Ala Gly His Arg Leu Tyr Gly Ile Ala Ile Asn Pro Asn Arg Val
225                 230                 235                 240
Phe Lys Val Asn Thr Asn Ala Tyr Tyr Glu Met Ser Gly Leu Glu Val
                245                 250                 255
Ser Phe Glu Glu Leu Arg Thr Phe Gly Gly His Asp Ala Lys Phe Ile
            260                 265                 270
Asp Ser Leu Gln Glu Asn Glu Phe Arg Leu Tyr Tyr Tyr Asn Lys Phe
        275                 280                 285

Lys Asp Ile Ala Ser Thr Leu Asn Lys Ala Lys Ser Ile Val Gly Thr
    290                 295                 300
Thr Ala Ser Leu Gln Tyr Met Lys Asn Val Phe Lys Glu Lys Tyr Leu
305                 310                 315                 320
Leu Ser Glu Asp Thr Ser Gly Lys Phe Ser Val Asp Lys Leu Lys Phe
                325                 330                 335
Asp Lys Leu Tyr Lys Met Leu Thr Glu Ile Tyr Thr Glu Asp Asn Phe
            340                 345                 350
Val Lys Phe Phe Lys Val Leu Asn Arg Lys Thr Tyr Leu Asn Phe Asp
        355                 360                 365
Lys Ala Val Phe Lys Ile Asn Ile Val Pro Lys Val Asn Tyr Thr Ile
    370                 375                 380
Tyr Asp Gly Phe Asn Leu Arg Asn Thr Asn Leu Ala Ala Asn Phe Asn
385                 390                 395                 400
Gly Gln Asn Thr Glu Ile Asn Asn Met Asn Phe Thr Lys Leu Lys Asn
                405                 410                 415
Phe Thr Gly Leu Phe Glu Phe Tyr Lys Leu Leu Gly Ser Ala Asp Leu
            420                 425                 430
Val Pro Arg Gly Ser Ala Asn Ser Ser Ser Val Asp Lys Leu Trp Ser
        435                 440                 445
His Pro Gln Phe Glu Lys Leu Glu His His His His His His
    450                 455                 460

<210> 18
<211> 464
<212> PRT
<213> Artificial Sequence

<223> "BoNT/A-Lc_ E325W/Y331W including His-tag"

<220>
<223> BoNT/A-Lc_ E325W/Y331W including His-tag

<400> 18
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Met Leu Leu Thr Ser Ile Val
            100                 105                 110
Arg Gly Ile Pro Phe Trp Gly Gly Ser Thr Ile Asp Thr Glu Leu Lys
        115                 120                 125
Val Ile Asp Thr Asn Cys Ile Asn Val Ile Gln Pro Asp Gly Ser Tyr
    130                 135                 140
Arg Ser Glu Glu Leu Asn Leu Val Ile Ile Gly Pro Ser Ala Asp Ile
145                 150                 155                 160
Ile Gln Phe Glu Cys Lys Ser Phe Gly His Glu Val Leu Asn Leu Thr
                165                 170                 175
Arg Asn Gly Tyr Gly Ser Thr Gln Tyr Ile Arg Phe Ser Pro Asp Phe
            180                 185                 190
Thr Phe Gly Phe Glu Glu Ser Leu Glu Val Asp Thr Asn Pro Leu Leu
        195                 200                 205
Gly Ala Gly Lys Phe Ala Thr Asp Pro Ala Val Thr Leu Ala His Glu
    210                 215                 220
Leu Ile His Ala Gly His Arg Leu Tyr Gly Ile Ala Ile Asn Pro Asn

225                 230                 235                 240
Arg Val Phe Lys Val Asn Thr Asn Ala Tyr Tyr Glu Met Ser Gly Leu
                245                 250                 255
Glu Val Ser Phe Glu Glu Leu Arg Thr Phe Gly Gly His Asp Ala Lys
            260                 265                 270
Phe Ile Asp Ser Leu Gln Glu Asn Glu Phe Arg Leu Tyr Tyr Tyr Asn
        275                 280                 285
Lys Phe Lys Asp Ile Ala Ser Thr Leu Asn Lys Ala Lys Ser Ile Val
    290                 295                 300
Gly Thr Thr Ala Ser Leu Gln Tyr Met Lys Asn Val Phe Lys Glu Lys
305                 310                 315                 320
Tyr Leu Leu Ser Trp Asp Thr Ser Gly Lys Trp Ser Val Asp Lys Leu
                325                 330                 335
Lys Phe Asp Lys Leu Tyr Lys Met Leu Thr Glu Ile Tyr Thr Glu Asp
            340                 345                 350
Asn Phe Val Lys Phe Phe Lys Val Leu Asn Arg Lys Thr Tyr Leu Asn
        355                 360                 365
Phe Asp Lys Ala Val Phe Lys Ile Asn Ile Val Pro Lys Val Asn Tyr
    370                 375                 380
Thr Ile Tyr Asp Gly Phe Asn Leu Arg Asn Thr Asn Leu Ala Ala Asn
385                 390                 395                 400
Phe Asn Gly Gln Asn Thr Glu Ile Asn Asn Met Asn Phe Thr Lys Leu
                405                 410                 415
Lys Asn Phe Thr Gly Leu Phe Glu Phe Tyr Lys Leu Leu Gly Ser Ala
            420                 425                 430
Asp Leu Val Pro Arg Gly Ser Ala Asn Ser Ser Ser Val Asp Lys Leu
        435                 440                 445
Trp Ser His Pro Gln Phe Glu Lys Leu Glu His His His His His His
    450                 455                 460

<210> 19
<211> 462
<212> PRT
<213> Artificial Sequence

<223> "BoNT/A-Lc_ 6465 _I303C_L310C including His-tag"

<220>
<223> BoNT/A-Lc_ 6465 _I303C_L310C including His-tag

<400> 19
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Lys
    50                  55                  60
Gln Val Pro Val Ser Tyr Tyr Asp Ser Thr Tyr Leu Ser Thr Asp Asn
65                  70                  75                  80
Glu Lys Asp Asn Tyr Leu Lys Gly Val Thr Lys Leu Phe Glu Arg Ile
                85                  90                  95
Tyr Ser Thr Asp Leu Gly Arg Met Leu Leu Thr Ser Ile Val Arg Gly
            100                 105                 110
Ile Pro Phe Trp Gly Gly Ser Thr Ile Asp Thr Glu Leu Lys Val Ile
        115                 120                 125
Asp Thr Asn Cys Ile Asn Val Ile Gln Pro Asp Gly Ser Tyr Arg Ser
    130                 135                 140
Glu Glu Leu Asn Leu Val Ile Ile Gly Pro Ser Ala Asp Ile Ile Gln
145                 150                 155                 160

Phe Glu Cys Lys Ser Phe Gly His Glu Val Leu Asn Leu Thr Arg Asn
                165                 170                 175
Gly Tyr Gly Ser Thr Gln Tyr Ile Arg Phe Ser Pro Asp Phe Thr Phe
            180                 185                 190
Gly Phe Glu Glu Ser Leu Glu Val Asp Thr Asn Pro Leu Leu Gly Ala
        195                 200                 205
Gly Lys Phe Ala Thr Asp Pro Ala Val Thr Leu Ala His Glu Leu Ile
    210                 215                 220
His Ala Gly His Arg Leu Tyr Gly Ile Ala Ile Asn Pro Asn Arg Val
225                 230                 235                 240
Phe Lys Val Asn Thr Asn Ala Tyr Tyr Glu Met Ser Gly Leu Glu Val
                245                 250                 255
Ser Phe Glu Glu Leu Arg Thr Phe Gly Gly His Asp Ala Lys Phe Ile
            260                 265                 270
Asp Ser Leu Gln Glu Asn Glu Phe Arg Leu Tyr Tyr Tyr Asn Lys Phe
        275                 280                 285
Lys Asp Ile Ala Ser Thr Leu Asn Lys Ala Lys Ser Cys Val Gly Thr
    290                 295                 300
Thr Ala Ser Cys Gln Tyr Met Lys Asn Val Phe Lys Glu Lys Tyr Leu
305                 310                 315                 320
Leu Ser Glu Asp Thr Ser Gly Lys Phe Ser Val Asp Lys Leu Lys Phe
                325                 330                 335
Asp Lys Leu Tyr Lys Met Leu Thr Glu Ile Tyr Thr Glu Asp Asn Phe
            340                 345                 350
Val Lys Phe Phe Lys Val Leu Asn Arg Lys Thr Tyr Leu Asn Phe Asp
        355                 360                 365
Lys Ala Val Phe Lys Ile Asn Ile Val Pro Lys Val Asn Tyr Thr Ile
    370                 375                 380
Tyr Asp Gly Phe Asn Leu Arg Asn Thr Asn Leu Ala Ala Asn Phe Asn
385                 390                 395                 400
Gly Gln Asn Thr Glu Ile Asn Asn Met Asn Phe Thr Lys Leu Lys Asn
                405                 410                 415
Phe Thr Gly Leu Phe Glu Phe Tyr Lys Leu Leu Gly Ser Ala Asp Leu
            420                 425                 430
Val Pro Arg Gly Ser Ala Asn Ser Ser Ser Val Asp Lys Leu Trp Ser
        435                 440                 445
His Pro Gln Phe Glu Lys Leu Glu His His His His His His
    450                 455                 460

<210> 20
<211> 463
<212> PRT
<213> Artificial Sequence

<223> "BoNT/A-Lc_ I138W_Q139E_140_D141N/S143KY144W  including His-tag  "

<220>
<223> BoNT/A-Lc_ I138W_Q139E_140_D141N/S143KY144W including His-tag

<400> 20
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Glu
    50                  55                  60
Ala Lys Gln Val Pro Val Ser Tyr Tyr Asp Ser Thr Tyr Leu Ser Thr
65                  70                  75                  80
Asp Asn Glu Lys Asp Asn Tyr Leu Lys Gly Val Thr Lys Leu Phe Glu
                85                  90                  95
Arg Ile Tyr Ser Thr Asp Leu Gly Arg Met Leu Leu Thr Ser Ile Val

            100                 105                 110
Arg Gly Ile Pro Phe Trp Gly Gly Ser Thr Ile Asp Thr Glu Leu Lys
        115                 120                 125
Val Ile Asp Thr Asn Cys Ile Asn Val Trp Glu Asn Gly Lys Trp Arg
    130                 135                 140
Ser Glu Glu Leu Asn Leu Val Ile Ile Gly Pro Ser Ala Asp Ile Ile
145                 150                 155                 160
Gln Phe Glu Cys Lys Ser Phe Gly His Glu Val Leu Asn Leu Thr Arg
                165                 170                 175
Asn Gly Tyr Gly Ser Thr Gln Tyr Ile Arg Phe Ser Pro Asp Phe Thr
            180                 185                 190
Phe Gly Phe Glu Glu Ser Leu Glu Val Asp Thr Asn Pro Leu Leu Gly
        195                 200                 205
Ala Gly Lys Phe Ala Thr Asp Pro Ala Val Thr Leu Ala His Glu Leu
    210                 215                 220
Ile His Ala Gly His Arg Leu Tyr Gly Ile Ala Ile Asn Pro Asn Arg
225                 230                 235                 240
Val Phe Lys Val Asn Thr Asn Ala Tyr Tyr Glu Met Ser Gly Leu Glu
                245                 250                 255
Val Ser Phe Glu Glu Leu Arg Thr Phe Gly Gly His Asp Ala Lys Phe
            260                 265                 270
Ile Asp Ser Leu Gln Glu Asn Glu Phe Arg Leu Tyr Tyr Tyr Asn Lys
        275                 280                 285
Phe Lys Asp Ile Ala Ser Thr Leu Asn Lys Ala Lys Ser Ile Val Gly
    290                 295                 300
Thr Thr Ala Ser Leu Gln Tyr Met Lys Asn Val Phe Lys Glu Lys Tyr
305                 310                 315                 320
Leu Leu Ser Glu Asp Thr Ser Gly Lys Phe Ser Val Asp Lys Leu Lys
                325                 330                 335
Phe Asp Lys Leu Tyr Lys Met Leu Thr Glu Ile Tyr Thr Glu Asp Asn
            340                 345                 350
Phe Val Lys Phe Phe Lys Val Leu Asn Arg Lys Thr Tyr Leu Asn Phe
        355                 360                 365
Asp Lys Ala Val Phe Lys Ile Asn Ile Val Pro Lys Val Asn Tyr Thr
    370                 375                 380
Ile Tyr Asp Gly Phe Asn Leu Arg Asn Thr Asn Leu Ala Ala Asn Phe
385                 390                 395                 400
Asn Gly Gln Asn Thr Glu Ile Asn Asn Met Asn Phe Thr Lys Leu Lys
                405                 410                 415
Asn Phe Thr Gly Leu Phe Glu Phe Tyr Lys Leu Leu Gly Ser Ala Asp
            420                 425                 430
Leu Val Pro Arg Gly Ser Ala Asn Ser Ser Ser Val Asp Lys Leu Trp
        435                 440                 445
Ser His Pro Gln Phe Glu Lys Leu Glu His His His His His His
    450                 455                 460

<210> 21
<211> 461
<212> PRT
<213> Artificial Sequence

<223> "BoNT/A-Lc_ 6465_ I138W_Q139E_140_D141N/S143KY144W including His-tag"

<220>
<223> BoNT/A-Lc_ 6465_ I138W_Q139E_140_D141N/S143KY144W including
His-tag

<400> 21
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg

35                  40                  45
Asp Thr Phe Thr Asn Pro Glu Glu Gly Asp Leu Asn Pro Pro Pro Lys
    50                  55                  60
Gln Val Pro Val Ser Tyr Tyr Asp Ser Thr Tyr Leu Ser Thr Asp Asn
65                  70                  75                  80
Glu Lys Asp Asn Tyr Leu Lys Gly Val Thr Lys Leu Phe Glu Arg Ile
                85                  90                  95
Tyr Ser Thr Asp Leu Gly Arg Met Leu Leu Thr Ser Ile Val Arg Gly
            100                 105                 110
Ile Pro Phe Trp Gly Gly Ser Thr Ile Asp Thr Glu Leu Lys Val Ile
        115                 120                 125
Asp Thr Asn Cys Ile Asn Val Trp Glu Asn Gly Lys Trp Arg Ser Glu
    130                 135                 140
Glu Leu Asn Leu Val Ile Ile Gly Pro Ser Ala Asp Ile Ile Gln Phe
145                 150                 155                 160
Glu Cys Lys Ser Phe Gly His Glu Val Leu Asn Leu Thr Arg Asn Gly
                165                 170                 175
Tyr Gly Ser Thr Gln Tyr Ile Arg Phe Ser Pro Asp Phe Thr Phe Gly
            180                 185                 190
Phe Glu Glu Ser Leu Glu Val Asp Thr Asn Pro Leu Leu Gly Ala Gly
        195                 200                 205
Lys Phe Ala Thr Asp Pro Ala Val Thr Leu Ala His Glu Leu Ile His
    210                 215                 220
Ala Gly His Arg Leu Tyr Gly Ile Ala Ile Asn Pro Asn Arg Val Phe
225                 230                 235                 240
Lys Val Asn Thr Asn Ala Tyr Tyr Glu Met Ser Gly Leu Glu Val Ser
                245                 250                 255
Phe Glu Glu Leu Arg Thr Phe Gly Gly His Asp Ala Lys Phe Ile Asp
            260                 265                 270
Ser Leu Gln Glu Asn Glu Phe Arg Leu Tyr Tyr Tyr Asn Lys Phe Lys
        275                 280                 285
Asp Ile Ala Ser Thr Leu Asn Lys Ala Lys Ser Ile Val Gly Thr Thr
    290                 295                 300
Ala Ser Leu Gln Tyr Met Lys Asn Val Phe Lys Glu Lys Tyr Leu Leu
305                 310                 315                 320
Ser Glu Asp Thr Ser Gly Lys Phe Ser Val Asp Lys Leu Lys Phe Asp
                325                 330                 335
Lys Leu Tyr Lys Met Leu Thr Glu Ile Tyr Thr Glu Asp Asn Phe Val
            340                 345                 350
Lys Phe Phe Lys Val Leu Asn Arg Lys Thr Tyr Leu Asn Phe Asp Lys
        355                 360                 365
Ala Val Phe Lys Ile Asn Ile Val Pro Lys Val Asn Tyr Thr Ile Tyr
    370                 375                 380
Asp Gly Phe Asn Leu Arg Asn Thr Asn Leu Ala Ala Asn Phe Asn Gly
385                 390                 395                 400
Gln Asn Thr Glu Ile Asn Asn Met Asn Phe Thr Lys Leu Lys Asn Phe
                405                 410                 415
Thr Gly Leu Phe Glu Phe Tyr Lys Leu Leu Gly Ser Ala Asp Leu Val
            420                 425                 430
Pro Arg Gly Ser Ala Asn Ser Ser Ser Val Asp Lys Leu Trp Ser His
        435                 440                 445
Pro Gln Phe Glu Lys Leu Glu His His His His His His
    450                 455                 460

<210> 22
<211> 461
<212> PRT
<213> Artificial Sequence

<223> "BoNT/A-Lc_ 6465_Q139E_P140_D141N/S143K    including His-tag"

<220>
<223> BoNT/A-Lc_ 6465_Q139E_P140_D141N/S143K including His-tag

<400> 22
Met Pro Phe Val Asn Lys Gln Phe Asn Tyr Lys Asp Pro Val Asn Gly
1               5                   10                  15
Val Asp Ile Ala Tyr Ile Lys Ile Pro Asn Ala Gly Gln Met Gln Pro
            20                  25                  30
Val Lys Ala Phe Lys Ile His Asn Lys Ile Trp Val Ile Pro Glu Arg
        35                  40                  45
Asp Thr Phe Thr Asn Pro Glu Glu Gly Asp Leu Asn Pro Pro Pro Lys
    50                  55                  60
Gln Val Pro Val Ser Tyr Tyr Asp Ser Thr Tyr Leu Ser Thr Asp Asn
65                  70                  75                  80
Glu Lys Asp Asn Tyr Leu Lys Gly Val Thr Lys Leu Phe Glu Arg Ile
                85                  90                  95
Tyr Ser Thr Asp Leu Gly Arg Met Leu Leu Thr Ser Ile Val Arg Gly
            100                 105                 110
Ile Pro Phe Trp Gly Gly Ser Thr Ile Asp Thr Glu Leu Lys Val Ile
        115                 120                 125
Asp Thr Asn Cys Ile Asn Val Ile Glu Asn Gly Lys Tyr Arg Ser Glu
    130                 135                 140
Glu Leu Asn Leu Val Ile Ile Gly Pro Ser Ala Asp Ile Ile Gln Phe
145                 150                 155                 160
Glu Cys Lys Ser Phe Gly His Glu Val Leu Asn Leu Thr Arg Asn Gly
                165                 170                 175
Tyr Gly Ser Thr Gln Tyr Ile Arg Phe Ser Pro Asp Phe Thr Phe Gly
            180                 185                 190
Phe Glu Glu Ser Leu Glu Val Asp Thr Asn Pro Leu Leu Gly Ala Gly
        195                 200                 205
Lys Phe Ala Thr Asp Pro Ala Val Thr Leu Ala His Glu Leu Ile His
    210                 215                 220
Ala Gly His Arg Leu Tyr Gly Ile Ala Ile Asn Pro Asn Arg Val Phe
225                 230                 235                 240
Lys Val Asn Thr Asn Ala Tyr Tyr Glu Met Ser Gly Leu Glu Val Ser
                245                 250                 255
Phe Glu Glu Leu Arg Thr Phe Gly Gly His Asp Ala Lys Phe Ile Asp
            260                 265                 270
Ser Leu Gln Glu Asn Glu Phe Arg Leu Tyr Tyr Tyr Asn Lys Phe Lys
        275                 280                 285
Asp Ile Ala Ser Thr Leu Asn Lys Ala Lys Ser Ile Val Gly Thr Thr
    290                 295                 300
Ala Ser Leu Gln Tyr Met Lys Asn Val Phe Lys Glu Lys Tyr Leu Leu
305                 310                 315                 320
Ser Glu Asp Thr Ser Gly Lys Phe Ser Val Asp Lys Leu Lys Phe Asp
                325                 330                 335
Lys Leu Tyr Lys Met Leu Thr Glu Ile Tyr Thr Glu Asp Asn Phe Val
            340                 345                 350
Lys Phe Phe Lys Val Leu Asn Arg Lys Thr Tyr Leu Asn Phe Asp Lys
        355                 360                 365
Ala Val Phe Lys Ile Asn Ile Val Pro Lys Val Asn Tyr Thr Ile Tyr
    370                 375                 380
Asp Gly Phe Asn Leu Arg Asn Thr Asn Leu Ala Ala Asn Phe Asn Gly
385                 390                 395                 400
Gln Asn Thr Glu Ile Asn Asn Met Asn Phe Thr Lys Leu Lys Asn Phe
                405                 410                 415
Thr Gly Leu Phe Glu Phe Tyr Lys Leu Leu Gly Ser Ala Asp Leu Val
            420                 425                 430
Pro Arg Gly Ser Ala Asn Ser Ser Ser Val Asp Lys Leu Trp Ser His
        435                 440                 445
Pro Gln Phe Glu Lys Leu Glu His His His His His His
    450                 455                 460